(19) Europäisches Patentamt
European Patent Office
Office européen des brevets

(11) EP 1 700 849 B1

(12) EUROPEAN PATENT SPECIFICATION

(45) Date of publication and mention of the grant of the patent:
**17.06.2009 Bulletin 2009/25**

(51) Int Cl.:
*C07D 229/02* (2006.01) *C07K 1/00* (2006.01)
*C12Q 1/00* (2006.01) *G01N 33/532* (2006.01)

(21) Application number: **05004954.3**

(22) Date of filing: **07.03.2005**

# (54) Photo-activatable amino acids

Photoaktivierbare Aminosäuren

Acides aminés photoactivables

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**13.09.2006 Bulletin 2006/37**

(73) Proprietor: **Max-Planck-Gesellschaft zur Förderung der Wissenschaften e.V.
Berlin (DE)**

(72) Inventors:
- **Thiele, Christoph
01328 Dresden (DE)**
- **Suchanek, Monika
01307 Dresden (DE)**

(74) Representative: **Vossius & Partner
Siebertstrasse 4
81675 München (DE)**

(56) References cited:

- **BRUNNER J: "NEW PHOTOLABELING AND CROSSLINKING METHODS" ANNUAL REVIEW OF BIOCHEMISTRY, PALTO ALTO, CA, US, vol. 62, 1993, pages 483-514, XP001068344 ISSN: 0066-4154**
- **IWASA JUNKICHI ET AL: "Synthesis of photoaffinity and tritium-labeled grayanotoxin via reductive amination" BIOSCIENCE BIOTECHNOLOGY AND BIOCHEMISTRY, vol. 58, no. 5, 1994, pages 972-973, XP009051791 ISSN: 0916-8451**
- **AHMED A R H ET AL: "ISOLATION AND PARTIAL PURIFICATION OF A MELANOCYTE-STIMULATING HORMONE RECEPTOR FROM B16 MURINE MELANOMA CELLS A NOVEL APPROACH USING A CLEAVABLE BIOTINYLATED PHOTOACTIVATED LIGAND AND STREPTAVIDIN-COATED MAGNETIC BEADS" BIOCHEMICAL JOURNAL, THE BIOCHEMICAL SOCIETY, LONDON, GB, vol. 286, 1992, pages 377-382, XP001033867 ISSN: 0264-6021**
- **FANG K ET AL: "A bifunctional photoaffinity probe for ligand/receptor interaction studies [14]" JOURNAL OF THE AMERICAN CHEMICAL SOCIETY 26 AUG 1998 UNITED STATES, vol. 120, no. 33, 26 August 1998 (1998-08-26), pages 8543-8544, XP002339631 ISSN: 0002-7863**

**EP 1 700 849 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

This invention relates to an amino acid based on a naturally occurring non-polar, non-aromatic α-amino acid, wherein said amino acid differs from the naturally occurring counterpart in that the side chain contains a diazirine ring.

In this specification, a number of documents is cited. The disclosure of these documents, including manufacturer's manuals, is herewith incorporated by reference in its entirety.

Protein-Protein interactions are the key to organizing cellular processes in space and time. Recently, systematic efforts[1, 2] have revealed that many, if not most, proteins function in the context of supramolecular complexes rather than as isolated entities. Interest in protein-protein interaction is not confined to basic research. Protein-protein interactions, functioning for example in viral fusion or in growth factor signaling are promising targets, for example for antiviral or anticancer drugs[3]. For the identification of interactions, various strategies are currently used. To screen for interaction partners, the yeast two hybrid system and some recent modifications (Thaminy et al. (2004), Lee & Lee (2004), Miller & Stagljar (2004)) are frequently used. An inherent disadvantage of these systems is the indirect read-out, i.e. via the activation of transcription of reporter genes, and the high frequency of false positive hits. For a more direct detection of protein-protein interaction, affinity-based methods such as co-immunoprecipitation are commonly used. Since these methods use cell lysates as a starting material, the detection of false positive interactions, resulting from the loss of spatial organization during the lysis procedure, is a problem. In addition, weakly interacting partners can be lost in the wash procedures.

To avoid these problems, chemical or photochemical cross-linking is used to covalently fix an interaction in a living cell, allowing detection under stringent conditions. Chemical cross-linking[4] employs moderately reactive bifunctional reagents, often directed against free amino groups, to generate covalent protein cross-links. Photo-crosslinking[5], in contrast, generates highly reactive intermediates in situ by irradiation of an inert precursor. The major advantage of photo-crosslinking lies in the increased specificity of cross-linking, due to the short lifetime of the excited intermediates. Photo-crosslinking requires the chemical introduction of a photo-activatable group into a protein, which initially limited the scope to small peptides that are amenable to total chemical synthesis (see e.g. Nassal (1984)). These peptides have been a useful tool in particular for the identification of peptide hormone receptors[6, 7]. Larger proteins became accessible with the in vitro synthesis of tRNAs bearing a photo-activatable amino acid[8, 9]. Introduction into specific positions was achieved by the use of tRNAs that suppress stop codons via in-vitro translation[10] and in vivo[11, 12]. Using engineered 'orthogonal' pairs of tRNAs and aminoacyl-tRNA-synthetases (aaRSs), various unnatural amino acids now can be incorporated into specific sites also in cultured mammalian cells[13-15]. While this is well suited for the production of engineered proteins, its application for general detection of protein-protein interaction in cell biology is limited by the complexity of the modified components (i.e. tRNA, aaRS, cDNA, unnatural amino acid) required and by the fact that only a single position in a single protein will bear the photoreactive group.

Iwasa et al. (Biosci. Biotech. Biochem., 58 (5), 972-973, 1994) describes photoaffinity-labeled L-lysine.

Ahmed et al. (Biochem. J. 286, 377-382, 1992) describes photoaffinity labeling for the isolation of a melanocyte-stimulating hormone receptor. As photoaffinity label 4-(1-azi-2,2,2-trifluoroethyl)benzoic acid (ATB) was used.

In Fang et al. (J. Am. Chem. Soc., 120, 8543-8544, 1998) a bifunctional photoaffinity probe comprising a diazirine ring has been described for ligand receptor interaction studies.

Altogether, Examples of incorporation of unnatural amino acids into proteins by the unmodified endogenous translation machinery are rare[34], usually confined to bacteria, and many of them are based on the particular promiscuity of bacterial MetRS[35, 36]. Other aaRSs are more stringent, and replacement is confined to nearly isosteric unnatural amino acids such as fluorinated derivatives of leucine, phenylalanine and tryptophane[37]. No example for an unnatural amino acid bearing a photoreactive group that would be incorporated into proteins by a eukaryotic cell with a genetically unmodified translational machinery has been reported so far.

In view of the limitations of the methods described in the prior art, the technical problem underlying the present invention was therefore the provision of alternative and/or improved means and methods for studying interactions involving proteins or (poly)peptides.

Accordingly, this invention relates to an amino acid based on a naturally occurring α-amino acid, said α-amino acid being selected from the group consisting of Leu, Met, Ile and Val, wherein said amino acid differs from the naturally occurring counterpart in that the side chain contains a diazirine ring, and wherein (a) in case of said naturally occurring counterpart being Met the carbon atom of the diazirine ring replaces the sulphur; and (b) in case of said naturally occurring counterpart not being Met the carbon atom of the diazirine ring is the penultimate carbon atom of the side chain, (i) wherein in case of said naturally occurring counterpart being Leu or Val one of the terminal methyl groups present in the side chain of said counterpart is absent in said amino acid.

The amino acids of the invention are designated as being based on a naturally occurring counterpart because, as further detailed below, the amino acids of the invention may replace one or more naturally occurring amino acids in (poly)peptides. The naturally occurring α-amino acids are alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu),

lysine (Lys), methionine (Met), phenylalanine (Phe), proline (Pro), serine (Ser), threonine (Thr), tryptophane (Trp), tyrosine (Tyr) and valine (Val). The predominant naturally occurring stereochemical configuration of the naturally occurring α-amino acids is the L-form. The naturally occurring amino acids may be partitioned into groups according to a variety of parameters. Based on the extent of electrostatic polarity, the may be grouped into non-polar and polar amino acids, wherein the polar amino acids may be further partitioned into those bearing a net charge and those being electrostatically neutral. Non-polar amino acids are Ala, Val, Leu, Ile, Met, Phe, Gly, Trp and Pro. Polar and overall neutral are Ser, Thr, Cys, Asn, Gln and Tyr. Tyr is very weakly acidic, but not charged at pH=7. Amino acids bearing a net charge at pH=7 are Lys, Arg, His, Asp and Glu. The terms "polar" and "non-polar" in general and in relation to amino acids is common in the art. Having regard to their chemical nature; the naturally occurring amino acids may be grouped into aliphatic (Ala, Gly, Ile, Leu, Pro and Val), aromatic (Phe, Trp and Tyr), acidic (Asp and Glu), basic (Arg, His and Lys), hydroxylic (Ser and Thr), sulphur-containing (Cys and Met) and amidic, i.e., amide group-containing (Asn and Gln) amino acids.
The term "(poly)peptide" as used herein describes proteins, fragments of proteins and synthetic molecules and refers to a group of molecules which comprise the group of peptides, consisting of up to 30 amino acids, as well as the group of polypeptides, consisting of more than 30 amino acids.
The term "based on" refers to the introduction of a diazirine ring and may include, besides the introduction of the ring, modifications of the structure to accommodate the ring. For example, if a carbon atom bears three substituents in the naturally occurring amino acid, rendering the carbon atom part of the diazirine ring necessitates removal of one of the substituents (for details see below).

The term "side chain" is well known in the art and refers to the moiety which differs between different naturally occurring amino acids. In the following generic formula of an amino acid, the side chain is designated with "R": $H_2N$-CHR-COOH. Accordingly, there is one side chain in a naturally occurring amino acid. As the amino acids of the invention are based on naturally occurring amino acids, they also exhibit a side chain.

The diazirine ring is a chemically inert, small hydrophobic precursor of highly reactive carbenes, that advantageously are capable of mediating cross-linking to any chemical group, even to non-functionalized C-H bonds. The mechanism of cross-linking by carbenes is well known in the art and described, for example, in Turro et al. (1987). Applications of carbene-crosslinking in biology are reviewed in Brunner (1993).

As shown in Example 3 enclosed herewith, the amino acids of the invention have the further advantage that they are non-toxic.

As stated above, said naturally occurring non-polar, non-aromatic α-amino acid is selected from the group consisting of Leu, Met, Ile and Val.

In a further preferred embodiment, the side chain is extended as compared to the naturally occurring counterpart by the insertion of one or more methylene groups such that the total number of carbon atoms does not exceed 8.

In a further preferred embodiment, said one or more methylene groups are inserted adjacent to a methylene group present in said naturally occurring counterpart.

Accordingly, in a preferred embodiment, a general formula of amino acids of the invention is as follows: $H_2N$-CH(COOH)-$(CH_2)_n$-C(N=N)-$(CH_2)_m$-$CH_3$, wherein n is an integer from 1 to 3, m is an integer from 0 to 3, the sum n+m is an integer from 1 to 4, and -C(N=N)- designates the diazirine ring.

If not specifically noted otherwise and/or specifically indicated in the respective formula, all formulae shown herein embrace both the L-form and the D-form of amino acids of the invention as well as of other compounds depicted and/or described herein exhibiting one ore more asymmetric centers.

In a further preferred embodiment, one or more hydrogen atoms attached to carbon atoms are substituted with fluorine.

In a more preferred embodiment, one or more methyl groups are replaced with trifluoromethyl.
The latter two embodiments relate to isosteric substitutions.

In a further preferred embodiment, the carbon atom of the diazirine ring is a carbon atom of the side chain of said naturally occurring counterpart. In other words, the diazirine ring is not a substituent added as a whole to the side chain, but instead what is formally being added are the two nitrogen atoms of the diazirine ring.

As stated above, according to the invention, (a) in case of said naturally occurring counterpart being Met the carbon atom of the diazirine ring replaces the sulphur; and (b) in case of said naturally occurring counterpart not being Met the carbon atom of the diazirine ring is the penultimate carbon atom of the side chain, wherein in case of said naturally occurring counterpart being Leu or Val one of the terminal methyl groups present in the side chain of said counterpart is absent in said amino acid. The above provides details regarding position of the diazirine ring, and, as indicated further above, regarding further modifications of the naturally occurring amino acid necessary to accommodate the diazirine ring and thereby to obtain the amino acid of the invention.

In a most preferred embodiment, said amino acid has one of the following formulae (I) to (III):

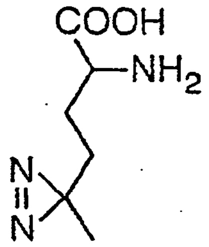

Formula (I)

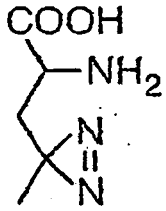

Formula (II)

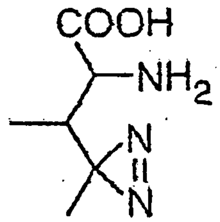

Formula (III)

The amino acid of formula (I) is also referred as photo-Met, the amino acid of formula (II) as photo-Leu, and the amino acid of formula (III) as photo-Ile. The nomenclature "photo-Xaa" has been adopted on the one hand because it is indicative of that naturally occurring amino acid, which the amino acid of the invention is intended to substitute, and on the other hand because is indicative of the property of being activatable by irradiation with light.

Considering formula (I) and (II) it is apparent that photo-Met can be considered as a derivative of norleucine and that photo-Leu is a derivative of norvaline, wherein said derivatives differ from norleucine and norvaline, respectively, by the introduction of a diazirine ring which is introduced such that the carbon atom of the diazirine ring is the penultimate carbon atom of norleucine and norvaline, respectively.

Accordingly, a distinction is to be made between the terms "based on" and "derivative of". Photo-Met, while being based on Met and intended to substitute Met in (poly)peptides, can also be considered a derivative of norleucine. Similarly, photo-Leu, while being based on Leu and intended to substitute Leu in (poly)peptides, can also be considered a derivative of norvaline.

In a further preferred embodiment, said amino acid is recognised and processed by a cognate wild-type aminoacyl-tRNA synthetase. Aminoacyl-tRNA synthetases, herein also abbreviated as aaRSs, are enzymes which attach am aminoacyl moiety to a tRNA molecule which in turn exhibits an anticodon complementary to the codon coding for that amino acid. Given the requirement of fidelity in mRNA translation, these enzymes have evolved to be highly specific, which applies in particular to the eukaryotic enzymes. In addition to a precise recognition and proofreading of the amino acid, the anticodon and other parts of the tRNA are recognised.

The amino acids of the invention, despite differing from their naturally occurring counterparts, are surprisingly recognised by the respective aminoacyl-tRNA synthetase as if or substantially as if they were the actual naturally occurring counterpart. The aminoacyl-tRNA synthetase performing the recognition and the processing of a particular amino acid, which comprises attaching to a matching tRNA molecule, is referred to as the cognate aminoacyl-tRNA synthetase.

Example 2 and furthermore Examples 4 to 6 enclosed herewith provide exemplary evidence that such recognition and processing of the amino acids of the invention by aminoacyl-tRNA synthetases occurs. Assays for the recognition of amino acids by aminoacyl-tRNA synthetases are known in the art, for example, the assay described in Hendrickson et al. (2000). An exemplary description of such an assay is given in the following.

Aminoacylation assays with photo-amino acids of the invention would be performed by mixing tRNA of the respective specificity, labelled photo-amino acid, preferably radioactively labelled photo-amino acid, and the respective aminoacyl-tRNA synthetase under appropriate conditions, followed by measurement of incorporation of label, preferably radioactive label into tRNA. Temperature may be between 0°C and 50°C and may depend on the optimal growth temperature of the organism the aminoacyl-tRNA synthetase originates from. Preferably, the temperature is 37°C.

Amino acid activation, which has to occur prior to aminoacylation may be evaluated by monitoring the photo-amino acid dependent ATP-pyrophosphate (PPi) exchange reaction. Temperature may be ambient temperature. The reaction mixture contains, for example, Tris-HCl (100 mM, pH 7.5), 2-mercaptoethanol (5mM), $MgCl_2$ (5 mM), ATP (2 mM), NaPPi (2 mM, 2000 cpm/nmol), bovine serum albumin (100 $\mu$g/ml), photo-amino acid of the invention (10 mM), and wild-type aminoacyl-tRNA synthetase or the appropriate mutant enzyme (100 nM). Aliquots will be removed at various times and quenched into a solution containing NaPPi (33 mM), 7% perchloric acid, and 8.3% (w/v) activated charcoal. The charcoal will be filtered through a 0.45 $\mu$m centrifugal filter (Z-Spin Plus, Gelman Sciences) and washed twice with a solution containing 10 mM NaPPi and 0.5% perchloric acid. Charcoal-absorbed [$^{32}$P]-ATP will be quantitated by scintillation counting of the filter and activated charcoal.

In a further preferred embodiment, said amino acid is processed by a wild-type ribosomal peptidyl transferase.

In a more preferred embodiment, said aminoacyl-tRNA synthetase and/or said peptidyl transferase is of eukaryotic origin. This embodiment particularly exploits the surprising feature of the amino acids of the invention which is the recognition and processing by the eukaryotic translation machinery, especially the eukaryotic aminoacyl-tRNA synthetases.

In a further preferred embodiment, said amino acid carries a protection group at the amino group and/or an activating group at the carboxy group. Suitable protection groups for the amino group include Boc (t-butyl-oxy-carbonyl) and Fmoc (Fluorenylmethyl-oxy-carbonyl). Suitable activating groups for the carboxy group include NHS (N-Hydroxysuccinimidyl) and Pfp (Pentafluorophenyl).

In a further preferred embodiment, said amino acid is isotopically labelled.

In a more preferred embodiment the isotopic label is selected from the group consisting of $^{2}H$, $^{3}H$ and $^{14}C$. Examples of this embodiment are provided in the Examples and Figures enclosed herewith.

The present invention also relates to a method of synthesising a (poly)peptide with one or more amino acids of the invention incorporated, comprising (a) allowing (poly)peptide synthesis to occur in an in vitro or cellular translation system in the presence one or more of said amino acids; and/or (b) performing chemical synthesis of said (poly)peptide, wherein one or more of said amino acids are used as starting material, and wherein said amino acid(s) may be protected and/or activated.

This embodiment provides two distinct means of producing a (poly)peptide with one or more amino acids of the invention incorporated, viz. enzymatic synthesis using a translation system and chemical synthesis, which may be used alternatively or in combination.

Said translation system may be an in vitro, i.e., a cell-free translation system. Examples of cell-free translation systems are lysates of wheat germs or reticulocytes. These systems are suitable for soluble proteins. Addition of dog pancreas microsomes permits the use of cell-free translation systems also for membrane proteins. Alternatively, said translation system is a cellular system. For that purpose, any living cell is envisaged.

Means and methods for chemical synthesis of (poly)peptides from amino acids as starting material in general are well known in the art. The chemical synthesis is a stepwise process yielding a growing (poly)peptide chain. For the purpose of coupling a further amino acid to the growing chain, specific protection ("blocking") and/or activation of functional groups is performed. Typically, and as further detailed herein above, the amino group is protected and the carboxy group is activated.

The phrasing "more amino acids" may refer to a plurality of amino acids of the same type, or a plurality of types of amino acids, wherein one or more amino acids of each type are intended. For example, a (poly)peptide with one or more amino acids of the invention incorporated may have 30-35% of the Met residues replaced with photo-Met, 10-20% of the Leu residues replaced with photo-Leu, and 10-20% of the Ile residues replaced with photo-Ile. Similarly, the phrasing "one amino acid", which is embraced by the words, "one or more amino acids", relates either to one single amino acid entity or to one type of amino acid, wherein in the latter case either all or a subset of the amino acids of said type are replaced with a corresponding amino acid of the invention.

With the amino acids of the invention the protein chemist has now the possibility to incorporate reactive functionality at previously unmodifiable sites. Surprisingly, this can be accomplished with amino acids of the invention without or without significant perturbation of the structure of the (poly)peptide.

Example 2 and Figure 2 provide evidence that incorporation of amino acids of the invention into proteins occurs when applying the method of the invention. Furthermore, the amino acids of the invention have the advantageous property of not or substantially not interfering with the function of the (poly)peptides or proteins the amino acids are incorporated into (see Example 4 and Figure 7).

In a preferred embodiment, said translation system comprises wild-type aminoacyl-tRNA synthetase as the only source of aminoacyl-tRNA synthetase activity.

It is a distinct advantage of the amino acids of the invention that there is no need for an artificially modified (engineered) aminoacyl-tRNA synthetase. It is well known in the art (and reviewed in the introductory part of this specification) that typically the structure of an aminoacyl-tRNA synthetase has to be modified in order to achieve recognition and processing of a non-natural amino acid.

In a further preferred embodiment, said translation system comprises wild-type ribosomal peptidyl transferase as the only source of peptidyl transferase activity.

In a more preferred embodiment, said translation system comprises in addition or alternatively to wild-type aminoacyl-tRNA synthetase one or more modified aminoacyl-tRNA synthetases, wherein said modified aminoacyl-tRNA synthetase(s) is/are specific for said amino acid(s).

Despite being it a distinct advantage of the amino acids of the invention that there is no need for engineered enzymes, it may be desirable for some applications to replace one or more wild-type aminoacyl-tRNA synthetases with modified version thereof, or to combine wild-type and modified variants. An example of a modified aminoacyl-tRNA synthetase is the mutant synthetase described in reference 11. A route to engineered synthetases which does not involve undue burden is provided in Santoro et al. (2002). As the method described in Santoro et al. (2002) involves mutagenesis and selection, an engineered synthetase obtainable thereby can not a priori be characterized by structural features.

In a more preferred embodiment, one or more tRNAs with modified anticodon(s) are provided, wherein said modified aminoacyl-tRNA synthetase(s) is/are capable of recognizing and processing said tRNA(s) with modified anticodon(s). The term "modified anticodon" refers to an anticodon within a particular tRNA which is changed such that the amino acid attached to said tRNA becomes part of the growing (poly)peptide chain at a position where it would not be incorporated if the anticodon were the anticodon present in the respective naturally occurring tRNA. Preferred modified anticodons are provided below. As noted above, the aminoacyl-tRNA synthetase also performs recognition of the anticodon. Therefore, generally it will be necessary to modify (engineer, mutate) the synthetase in order to ensure that a tRNA with a modified anticodon is recognised and processed. Methods therefore are known in the art and cited above.

In a yet more preferred embodiment, said modified anticodon is complementary to the amber stop codon (UAG) or complementary to a four-base codon.

In a preferred embodiment of the method of the invention, said cellular translation system is comprised in a eukaryotic cell line.

In a further preferred embodiment of the method of the invention, the naturally occurring counterpart(s) of the amino acid(s) of the invention is/are absent. The absence of the naturally occurring counterpart(s) ensures that competing of the amino acid(s) of the invention with their naturally occurring counterpart(s), which would interfere with incorporation, does not occur.

In a further preferred embodiment of the method of the invention, said amino acid(s) is/are provided at a concentration of about 0.1 mM to about 10 mM, more preferably between 1 mM and 5 mM.

Also preferred are concentrations which are between the about 2- and about 20-fold concentration of the naturally occurring counterpart of the amino acid under conditions where no amino acid(s) of the invention is/are provided. In other words, said conditions (and concentrations of the naturally occurring counterpart(s)) are the conditions of (poly) peptide synthesis (and concentration(s) of naturally occurring amino acid(s)) commonly used in the art, i.e., where amino acids of the invention are not used. About 2- to about 20-fold elevated concentration are preferred, as the wild-type aminoacyl-tRNA synthetases, despite recognising and processing the amino acids of the invention, may do so with a lower affinity and/or catalytic rate.

Also preferred are concentrations leading to an overall incorporation rate and an overall incorporation rate, wherein said overall incorporation rate is between about 0.1% and about 5% (mole of amino acid(s) of the invention incorporated / mole of total amino acid in the (poly)peptide). The term "overall incorporation rate" relates to a measure of incorporation across all photo-amino acids of the invention used.

The present invention also relates to a method for cross-linking interacting molecules in a biological system comprising a translation system, wherein at least one of said interacting molecules is a (poly)peptide, the method comprising the steps of: (a) allowing (poly)peptide synthesis to occur in said biological system in the presence of one or more amino acids according to the invention; and (b) irradiating said biological system such that the diazirine ring of said amino acid undergoes photolysis.

The term "cross-linking" refers to the formation of one or more covalent bonds between said interacting molecules. Said cross-linking occurs after irradiating and in the immediate proximity of the photo-activatable amino acid of the invention. It occurs via photolysis of the diazirine ring, concomitant carbene formation and reaction of the carbene with any chemical group in the immediate proximity thereof. Thereby, a covalent fingerprint of the interaction is obtained.

The term "biological system" as used herein refers to a system comprising one or more biological molecules under conditions that allow molecular interactions, if any, to occur. The term "biological molecule" designates a molecule originating from an organism or a derivative obtained therefrom, wherein at least one biological molecule or derivative thereof is a (poly)peptide. Derivatives which are envisaged in particular are (poly)peptides comprising one or more amino acids according to the invention. If one or more membrane (poly)peptides or membrane proteins are comprised in the system, they may be solubilized as parts of micelles or membrane preparations. To the extent reference is made to a method for cross-linking of the invention, it is understood that said biological system furthermore comprises a translation system. The translation system may be a eukaryotic or a prokaryotic translation system. The term "translation system" is further described herein above.

Conditions allowing molecular interactions, enzymatic reactions and/or chemical reactions involving biological molecules to occur are known to the skilled person. Exemplary conditions comprise buffered aqueous solutions. The temperature may be, for example, ambient temperature. Alternatively, and if biological molecules originating from psychrophilic, thermophilic or hyperthermophilic organisms are comprised in the biological system, the preferred temperature interval may be centered around the optimal growth temperature of the source organism. A particularly preferred temperature is 37°C. Alternatively, the temperature may be ambient temperature. For biological processes and/or interactions to occur, the presence of further substances may be necessary, including sodium chloride and other salts, trace elements, amino acids, vitamins, growth factors, ubiquitous cofactors such as ATP or GTP. Said further substances may either be added individually or provided in complex mixtures such as serum or cell extracts. These and further accessory substances are well known in the art as are concentrations suitable for biological assays.

Conditions that allow molecular interactions to occur comprise physiological conditions. Physiological conditions in accordance with the present invention may vary significantly, for example when comparing the interior of a cell to the extracellular space. Exemplary intracellular conditions comprise 14 mM $Na^+$, 140 mM $K^+$, $10^{-7}$ mM $Ca^{2+}$, 20 mM $Mg^{2+}$,

4 mM $Cl^-$, 10 mM $HCO_3^-$, 11 mM $HPO_4^{2-}$ and $H_2PO_4^-$, 1 mM $SO_4^{2-}$, 45 mM phosphocreatine, 14 mM carnosine, 8 mM amino acids, 9 mM creatine, 1.5 mM lactate, 5 mM ATP, 3.7 mM hexose monophosphate, 4 mM protein and 4 mM urea. Exemplary interstitial conditions comprise 140 mM $Na^+$, 4 mM $K^+$, 1.2 mM $Ca^{2+}$, 0.7 mM $Mg^{2+}$, 108 mM $Cl^-$, 28.3 mM $HCO_3^-$, 2 mM $HPO_4^{2-}$ and $H_2PO_4^-$, 0.5 mM $SO_4^{2-}$, 2 mM amino acids, 0.2 mM creatine, 1.2 mM lactate, 5.6 mM glucose, 0.2 mM protein and 4 mM urea.

Buffers are well known in the art and the skilled person is aware of appropriate buffers in dependency of the biological molecules being studied. Common buffers comprise ($pK_a$ values in brackets) $H_3PO_4$ / $NaH_2PO_4$ ($pK_{a,1}$ = 2.12), Glycine ($pK_{a,1}$ = 2.34), Acetic acid (4.75), Citric acid (4.76), MES (6.15), Cacodylic acid (6.27), $H_2CO_3$ / $NaHCO_3$ ($pK_{a,1}$ = 6.37), Bis-Tris (6.50), ADA (6.60), Bis-Tris Propane ($pK_{a,1}$ = 6.80), PIPES (6.80), ACES (6.90), Imidazole (7.00), BES (7.15), MOPS (7.20), $NaH_2PO_4$ /$Na_2HPO_4$ ($pK_{a,2}$ = 7.21), TES (7.50), HEPES (7.55), HEPPSO (7.80), Triethanolamine (7.80), Tricine (8.10), Tris (8.10), Glycine amide (8.20), Bicine (8.35), Glycylglycine ($pK_{a,2}$ = 8.40), TAPS (8.40), Bis-Tris Propane ($pK_{a,2}$ = 9.00), Boric acid ($H_3BO_3$ / $Na_2B_4O_7$) (9.24), CHES (9.50), Glycine ($pK_{a,2}$ = 9.60), $NaHCO_3$ /$Na_2CO_3$ ($pK_{a,2}$ = 10.25), CAPS (10.40) and $Na_2HPO_4$ / $Na_3PO_4$ ($pK_{a,3}$ = 12.67).

Furthermore, ionic strength may be adjusted, e.g., by the addition of sodium chloride and/or potassium chloride. The concentration of sodium chloride is between 0 and 2 M, preferably between 100 and 200 mM. Examples comprise PBS (phosphate buffered saline) containing 1.37 M NaCl, 27 mM KCl, 43 mM $Na_2HPO_4 \cdot 7H_2O$ and 14 mM $KH_2PO_4$ in the 10-fold aqueous stock solution, which is adjusted to pH 7.3; SSC containing 3 M NaCl and 0.3 M sodium citrate in 20-fold aqueous stock solution, which is adjusted to pH 7.0; and STE (Saline Tris EDTA) containing 10 mM Tris base, 10 mM NaCl and 1mM ETA (acid). Alternatively, sodium chloride is absent from the buffer preparation. Examples for common buffer preparations without sodium or potassium chloride are TAE (Tris acetate EDTA) containing 2 M Tris acetate and 0.1 M EDTA in the 50-fold aqueous stock solution at pH 8.5; TBE (Tris borate EDTA) containing 0.89 M Tris base, 0.89 M Boric acid and 0.02 M EDTA in the 10-fold aqueous stock solution at pH 8.0; and TE (Tris EDTA) containing 10 mM Tris base and 1 mM EDTA (acid) at pH 7.5.

The synthesis of the (poly)peptide may occur already in the presence of one or more molecules interacting therewith. Alternatively, the method may involve the further step of bringing said (poly)peptide, once synthesised, into contact with one or more test molecules or interacting molecules, wherein said further step is effected between steps (a) and (b).

As described above, the photo-activatable amino acids of the invention are efficiently incorporated into proteins by mammalian cells without the need of modified tRNAs or aaRSs, and yield specific cross-linking after photo-activation (see Example 5). In view of the fact that no modification of the translation system is needed, the method for cross-linking of the invention is characterized by particular ease of application and is also referred to as "feed-and-flash" strategy, i.e., it is characterized by the essential steps of providing the amino acids of the invention, for example to a translation system, and irradiation. As demonstrated in Example 5, the method of the invention is applicable to both endogenous and transfected proteins.

The present invention also relates to a method for cross-linking interacting molecules, wherein at least one of said interacting molecules is a (poly)peptide, the method comprising the steps of: (a) performing chemical synthesis of said (poly)peptide, wherein one or more amino acids of the invention are used as starting material, and wherein said amino acid(s) may be protected and/or activated; (b) bringing said (poly)peptide into contact with one or more test molecules; and (c) irradiating the system obtained in step (b) such that the diazirine ring of said amino acid undergoes photolysis.

This embodiment relates to a method of cross-linking, wherein at least one of the molecules to be cross-linked is obtained by chemical synthesis, and wherein said molecule is a (poly)peptide. It is also envisaged that said (poly) peptide is not obtained by pure chemical synthesis, but by a combination of synthesis effected with a translation system described further herein above and chemical synthesis. Chemical synthesis of (poly)peptides as such is well known in the art and described herein above.

The term "test molecule" or "interacting molecule" as used herein refers to any molecule, which may be proteinaceous or not.

The step of bringing said (poly)peptide into contact with one or more test molecules may be effected either subsequently to step (a) or simultaneously. In the latter case, said test molecules are already present during the synthesis of said (poly)peptide.

Accordingly, the photo-activatable amino acids of the invention are not restricted to uses for the above described "feed-and-flash" strategy. Due to the extraordinary chemical stability of aliphatic diazirines, all three amino acids can be used in solid phase (poly)peptide synthesis to extend the repertoire of available photo-crosslinking amino acids, which are mostly derivatives of phenylalanine (for example described in Nassal (1984)).

The above methods for cross-linking combine the broad range of chemical cross-linking with the superior specificity of photo-crosslinking employing the photo-activatable amino acids of the invention which have unique similarity to natural amino acids.

It should be noted that covalent cross-linking with a zero-distance photo-crosslinker like the amino acids of the

invention is the only way to prove a direct interaction between two proteins of a multi-protein complex apart from crystallographic data, which are not available for the vast majority of membrane proteins.

In a preferred embodiment, the method for cross-linking comprises the further step(s) of (d) determining the identity of the interacting molecules; and/or (e) determining the specific positions of said interacting molecules involved in the interaction of said interacting molecules.

Determining the identity of the interacting molecules may be effected by any method known in the art. Examples include the use of antibodies and mass spectrometry. Specifically, the cross-linked complex obtained by the method of the invention is subjected to mass spectrometry under conditions where fragmentation occurs. Fragments comprising an amino acid of the invention and involved in a cross-link exhibit a mass distinct from the mass of corresponding fragments comprising an amino acid of the invention but not being involved in cross-link, and also distinct from the mass of corresponding fragments comprising the naturally occurring counterpart of said amino acid of the invention. These differences in mass may also be used to determine the specific positions of said interacting molecules involved in said interaction. Further methods for determining the identity of the interacting molecules include immunoprecipitation and N-terminal sequencing. Exemplary determinations of interactions are shown in Example 6 enclosed herewith.

In a preferred embodiment, said irradiating is effected with UV light of a wavelength between about 290 and about 380 nm. More preferably, said wavelength is greater than 310 nm.

Half times $t_{1/2}$ for photoactivation depend on the intensity of light of the lamp used and on the geometry of the irradiation setup. Half times $t_{1/2}$ may typically be in the range between about 5 and about 60 seconds, for example 15 to 20 sec. Preferred irradiation times are between about 0.1 and 100 times $t_{1/2}$, more preferred between 1 and 10 times $t_{1/2}$. Accordingly, preferred irradiation times, in particular when conditions as described in Example 1 are used, are between about 1 and about 3 min for experiments with living cells. For example, irradiation may be effected for 60, 70, 80, 90, 120, 150 or 180 seconds. However, also shorter irradiation times, for example, 0.1, 1, 2, 5 or 10 seconds, are envisaged. Under all these conditions, cell viability is unaffected or substantially unaffected; reduced viability became apparent only at irradiation times significantly longer than 10 minutes (see also Example 1). Accordingly, also irradiation times up to 10 minutes, for example, 4, 5, 6, 7, 8, 9 or 10 minutes, under certain conditions also longer 10 minutes are envisaged.

In a further preferred embodiment of the methods of the invention, at least one of the (poly)peptides is a membrane protein or membrane (poly)peptide.

There is a greater likelihood of a non-polar amino acid being involved in an interaction, when the (poly)peptide the amino acid is part of is a membrane protein or membrane (poly)peptide. As the amino acids of the invention are based on non-polar naturally occurring amino acids, their use in the study of interactions involving membrane proteins is particularly advantageous. Furthermore it is of note that prior to the disclosure of the present invention, the determination or confirmation of a direct interaction within a cell membrane present a particular challenge.

However, and as non-polar amino acids are also involved in interactions of soluble proteins, it is also envisaged to apply the methods of the invention to soluble (poly)peptides as shown in Figure 5 (Rpt1 and Rpt4 are soluble cytosolic proteins).

In a more preferred embodiment a plurality of interactions is being determined, thereby providing one or more networks of biological interactions.

It is well known that molecular interactions in biological systems are not confined to pairwise interactions. Rather, a given first molecule may interact with a plurality of second molecules simultaneously and/or subsequently, thereby giving rise to a network. A network may be represented by a graph comprising nodes and edges. Nodes and edges in turn represent molecules and interactions, respectively. If a biological system is subjected to a method for cross-linking according to the invention, several such networks may be studies, elucidated and/or confirmed. Such networks or pathway may be, for example, metabolic networks or signal transduction networks.

The present invention also relates to a kit comprising (a) one or more amino acids according to the invention; and (b) a manual with instructions for performing any method of the invention. The kit may furthermore comprise growth media which are free of the naturally occurring counterpart(s) of the photo-amino acid(s) of the invention to be used. Said medium may furthermore comprise serum dialyzed against PBS.

In a preferred embodiment, the kit furthermore comprises media devoid of one or more of the naturally occurring counterpart(s) of the amino acid(s) of the invention.

Also provided is a method of synthesizing an amino acid of formula (I) as defined above, comprising the step of reacting 4,4'-azi-pentanal with $NH_3$, $NH_4Cl$ and NaCN. This embodiment relates to the Strecker synthesis for amino acids of the invention. Strecker synthesis as such is known in the art as are variants thereof yielding pure enantiomers.

Furthermore, a method of synthesizing an amino acid of formula (I) is provided, comprising the steps of: (a) reacting 5-oxo-hexanoic acid with $NH_3$, $NH_2OSO_3H$ and $I_2$; (b) brominating the 5,5'-azi-hexanoic acid obtained in step (a); and (c) aminolysis of the 2-bromo-5,5'-azi-hexanoic acid obtained in step (b).

Analogously, a method of synthesizing an amino acid of formula (II) as defined above is provided, comprising the steps of: (a) reacting 4-oxo-pentanoic acid with $NH_3$, $NH_2OSO_3H$ and $I_2$; (b) brominating the 4,4'-azi-pentanoic acid

obtained in step (a); and (c) aminolysis of the 2-bromo-4,4'-azi-pentanoic acid obtained in step (b).

Analogously, a method of synthesizing an amino acid of formula (III) as defined above is provided, comprising the steps of: (a) reacting 3-methyl-4-oxo-pentanoic acid with $NH_3$, $NH_2OSO_3H$ and $I_2$; (b) brominating the 3-methyl-4,4'-azi-pentanoic acid obtained in step (a); and (c) aminolysis of the 2-bromo-3-methyl-4,4'-azi-pentanoic acid obtained in step (b).

Step (a) of the methods of synthesizing of the invention generically comprises the conversion of a ketone into a Schiff base using $NH_3$, followed by the in situ formation of a diaziridine by the addition of hydroxylamine-O-sulfonic acid, and the oxidation of the diaziridine to a diazirine with iodine (see also Church & Weiss (1970)). The method of synthesis according to the invention are exemplified in Example 7 enclosed herewith.

In a preferred embodiment the method of the invention comprises the further step of separating the L-form of said amino acid from the D-form. It is well known in the art how to effect said step of separating the L-form from the D-form. Furthermore, it is exemplified in Example 7 enclosed herewith.

The Figures show:

**Figure 1:** Photoreactive amino acids of the invention. (A) The structures of photo-Ile, photo-Leu and photo-Met are shown in comparison to the natural amino acids Ile, Leu and Met. (B) Enantiomeric and radiochemical purity of photo-Ile, photo-Leu and photo-Met. DL-mixtures or pure enantiomers as indicated were separated by TLC on a chiralplate. Start and solvent front were at the bottom and top of the image, respectively. Amino acids were detected by ninhydrin staining (lanes1-7) or autoradiography (lane 8).

**Figure 2:** Incorporation of [1-$^{14}$C]photo-Met into proteins. (a) COS7 cells were grown in the absence (lanes 2 and 4) or presence (lanes 1 and 3) of [1-$^{14}$C]photo-Met. Proteins were separated on SDS-PAGE, followed by Coomassie-blue staining (lanes 1 and 2) and fluorography (lanes 3 and 4). (B) Precipitated proteins were digested with pronase. An excess of unlabeled Glu and photo-Met was added followed by heating with 7 N HCl. Amino acids were resolved by chromatography on Dowex 50WX8. Column fractions were assayed for radioactivity, for absorption at 350 nm to follow photo-Met, and for amino groups using the fluorescamine assay. Values are corrected for background and normalized to the most intense peak. Total recovery of radioactivity in the column eluate was 85%.

**Figure 3:** Photo-crosslinking induced by photo-amino acids. COS7 cells (panels a,b) or HeLa cells (panel c) were grown in LM-4 (panel a) or LM-2 (panels b,c) in the absence or presence of L-photo-Leu (pL), L-photo-Met (pM) or DL-photo-Ile (pl) or mixtures of them (pLM or pLMI) followed by UV irradiation (+) or no irradiation (-). cross-linked products formed after UV-irradiation were detected by SDS-PAGE followed by western blotting using (a) anti-caveolin 1 to detect overexpressed caveolin 1, (b) anti-stomatin to detect endogenous stomatin or (c) anti-EEA1 to detect endogenous EEA1. In panel b, lanes 8 and 9 show cross-linking with two different concentrations of the chemical cross-linker DSS for comparison of cross-linking efficiency. In panel c, photocrosslinking of endogenous EEA1 by photo-Leu and photo-Met is compared to chemical cross-linking using DSS or para-formaldehyde (PFA) as indicated. Note that chemical cross-linking leads to protein aggregates that are not resolvable by SDS-PAGE while photo-crosslinking identifies more than six discrete cross-linked bands.

**Figure 4:** Specificity of incorporation and cross-linking by photo-Met and photo-Leu. COS7 cells were transfected with HA-tagged human PGRMC1 and were grown in LM-4 in the absence or presence of photo-Leu (A) or photo-Met (B) with addition of 3 mM of the indicated competing amino acid. cross-linked products formed after UV-irradiation were detected by SDS-PAGE followed by western blotting against the HA-epitope. (C) COS7 cells were grown in the presence of 1.7 mM [1-$^{14}$C]photo-Met, a constant trace amount of [$^{3}$H]Phe and 3 mM of competing unlabeled amino acids as indicated in the legend. Protein was precipitated and protein-bound radioactivity was determined by scintillation counting. The plot shows the amount of incorporated [1-$^{14}$C]photo-Met, normalized to protein synthesis as determined by incorporated [$^{3}$H]Phe. The control value obtained in the absence of competing amino acid (con) is set to 100. Data are average $\pm$ SD of triplicate determinations.

**Figure 5:** Photocrosslinking shows specific interactions of proteasome subunits in vivo. HeLa cells were grown in LM-2 the presence of photo-Leu and photo-Met followed by UV irradiation (+) or no irradiation (-). Cytosolic fractions were subjected to SDS-PAGE and proteins transferred to nitrocellulose. Lanes were cut into single stripes, incubated with primary (anti-hRpt1 or anti-hRpt4) and secondary antibodies and were reassembled in the original order before ECL detection. Note that Rpt4 gives three discrete cross-linked bands, none of which contains Rpt1. A very long exposure shows a cross-linked band of Rpt1 migrating slower than the three cross-linked bands formed by Rpt4 (not shown).

**Figure 6:** Direct interaction in vivo between Insig-1 and PGRMC1 demonstrated by photocrosslinking. a) COS7

cells were transfected with HA-tagged PGRMC1 and myc-tagged Insig-1 and were grown in LM-4 in the presence or absence of photo-Met. Cells were UV-irradiated or not irradiated as indicted, lysed and subjected to immunoprecipitation (IP) with anti-HA or anti-myc antibodies as indicated. Immunoprecipitated proteins were separated by SDS-PAGE and probed (WB) for the presence of cross-links with anti-myc antibody or anti-HA antibody as indicated. The small difference in apparent molecular weight of the cross-link is due to different percentage of SDS-PAGE gels used. The presence of uncrosslinked Insig-1 in the left lanes indicates non-covalent co-immunoprecipitation. The strong band in lanes 3 and 4 (IgG-HC) is crossreacting IgG heavy chain.
b) COS7 cells were transfected with tagged proteins as indicated, cross-linked and subjected to immunoprecipitation followed by SDS-PAGE/western blotting as above. Note that SCAP forms a high molecular weight cross-link, likely a dimer, and Insig1 cross-links to PGRMC1, while Insig-1 does not cross-link to TRP-1-ER that is also localized to the endoplasmic reticulum.

**Figure 7:** Localization and fluorescence of EGFP-Rab5, EGFP-Rab5QL and EGFP-Rab5SN are not affected by the presence of photo-amino acids in the growth medium. COS7 cells were transfected with EGFP-fusion constructs of the small GTPase Rab5 and of mutated Rab5 that is constitutively activated (GFP-Rab5QL) or dominant negative (GFP-Rab5SN). Four hours after transfection cells were switched to various different media without and with the addition of photo-amino acids as indicated, and kept in these for further 20 h. Cells were fixed with 1% paraformaldehyde, mounted in moviol with antifade added, and subjected to confocal laser scanning microscopy using a Zeiss LSM at 488 nm excitation. Note that both EGFP-fluorescence intensity and protein localization were unaffected by the presence of either of the photo-amino acids (rows d-f).

**Figure 8:** 1 H-NMR spectrum of photo-Met.

**Figure 9:** 13C-NMR spectrum of photo-Met.

**Figure 10:** 1 H-NMR spectrum of photo-Leu.

**Figure 11:** 13C-NMR spectrum of photo-Leu.

**Figure 12:** 1 H-NMR spectrum of photo-Ile.

**Figure 13:** 13C-NMR spectrum of photo-Ile.

**Figure 14:** UV spectra of photo-Met, photo-Leu and photo-Ile.

The following examples illustrate the invention but should not be construed as being limiting.

**Example 1**

**Material and Methods**

Biochemical methods

Antibodies used in this study were mouse monoclonal antibody (mab) anti-HA clone F-7, rabbit polyclonal anti-HA Y-11, mouse mab anti-myc clone 9E10, rabbit polyclonal anti-myc A-14, rabbit polyclonal anti-caveolin-1 N-20, all from Santa Cruz, mab GARP50 against stomatin[22], mabs p42-23 against human Rpt4 and MSS1-104 against human Rpt1 (both from Biomol), rabbit polyclonal antiserum against EEA1[23].

Expression constructs

A cDNA coding for human caveolin-1 with a C-terminal VSVG-tag[42] was a kind gift of K. Simons. A cDNA for human PGRMC1 (Unigene Hs. 90061) was obtained by PCR from an EST clone (IMAGE 3863377) and was cloned into a pcDNA 3.1Hygro(+) vector with HindIII and SpeI restriction sites. Into the resulting construct a triple HA-tag (3x YPYDVPDYA) was inserted at the C-terminus of the protein before the stop codon. SCAP-3HA was constructed as above using IMAGE clone 5271717 and BamHI and SpeI restriction sites of the vector. TRP1-3HA-ER was constructed as above using IMAGE clone 3052338 and EcoRI and XhoI restriction sites of the vector. An additional ER retrieval signal was added between 3HA tag and STOP codon. A cDNA for human Insig-1 was obtained by PCR from an EST clone (IMAGE 4545505) and was cloned into a pcDNA 3.1Hygro(+) vector with HindIII and SpeI restriction sites. Into the

resulting construct sequential myc tags (9 x EQKLISEEDL) were inserted at the C-terminus of the protein before the stop codon. All products were verified by sequencing. Transfection was performed using Lipofectamine 2000 (Invitrogen) according to manufacturers instructions.

Expression and localization to the endoplasmatic reticulum of PGRMC1, SCAP, Insig-1 and TRP-1-ER constructs was verified by western blotting and confocal immunofluorescence microscopy, respectively (data not shown).

Cell culture and labeling with photo-amino acids

COS7 (monkey kidney) cells were grown in DMEM high glucose (Gibco no. 11995; with L-glutamine and Na-pyruvate) + 10% FCS. Typically, 3cm2 of cells are needed for a cross-linking experiment with detection by western blotting. At about 70%, medium was replaced by LM-4 (DMEM lacking Met, Leu, Ile and Val and phenol red, supplemented with 5% dialyzed FCS) or by LM-2 (LM-4 + 1 mM of each Ile and Val). Photo-amino acids were added to a final concentration of 4 mM photo-Leu and photo-Ile, 1.7mM photo-Met and cells were cultivated for further 22 h followed by washing with PBS and UV-irradiation using a 200 W high pressure mercury lamp (Oriel Photomax) with a glass filter to remove wavelengths shorter 310 nm. The samples were placed at a distance of 38 cm to the light source, corresponding to a illuminated area of 105 mm diameter. Under these conditions, the $t_{1/2}$ for photoactivation, determined by following the diazirine absorption peak at 340 to 350 nm, was 20 s. We used an irradiation time 1-3 min for experiments with living cells. Under these conditions, cell viability was unaffected; reduced viability became apparent only at irradiation times significantly longer than 10 minutes (see Table 3). Lysis of cells, SDS-PAGE and western blotting were done as described[33]. For immunoprecipitation, cells were lysed in 20mM Hepes/NaOH pH 7.4, 100mM NaCl, 5mM EDTA, 1% Triton X 100, 0.5 % sodium deoxycholate, the lysate was cleared with protein-A beads and incubated with beads that were preincubated with polyclonal anti-myc or anti-HA antibody. Beads were collected and washed three times each with 20mM Hepes/NaOH pH 7.25, 0.5% CHAPS and with 100 mM Tris-HCl pH 8.3, 150 mM NaCl, 2 mM EDTA, 0.1 % w/v SDS, 0.5% w/v Nonidet P40, 0.5% w/v sodiumdeoxycholate. Proteins were eluted from beads by heating with reducing SDS-PAGE sample buffer.

For the experiment shown in Fig. 5, Hela cells were grown in LM-2 +photo-Met + photo-Leu and UV irradiated or not. Cells were washed with PBS and HB (15 mM Hepes/NaOH pH 7.4 + 0.25 M sucrose), scraped into HB+protease inhibitors and were homogenized in an EMBL cell cracker (on ice, 12 micrometer clearance, 10 strokes). Homogenates were sequentially centrifuged at 3000 g for 5 min and at 60000 g for 1 h. Proteins in the final supernatants were precipitated by chloroform/methanol and subjected to SDS-Page followed by western blotting.

Chemical cross-linking

COS7 or HeLa cells were grown in LM-2, washed three times with PBS and were incubated with 1-2 mM of DSS or with 1% para-formaldehyde, both in PBS for 30 min at room temperature followed by quenching for 15 min with 10 mM Tris pH 8.6 or 5 mM glycine, respectively. Cell lysis and processing for SDS-PAGE/western blotting was as described above.

Analysis of incorporation of radioactive amino acids into protein

COS7 cells were grown for 20 h in LM-2 containing radiolabeled amino acids ([1-$^{14}$C]photo-Met and [$^{3}$H]Phe). Cells were washed 6 times with PBS and lysed in 0.5% Triton X-100, 0.2% sodium deoxycholate. Aggregated DNA was removed, protein was precipitated[43], the pellet was redissolved in 0.3 ml 0.5% SDS and subjected to a second round of precipitation. More than 99% of total radioactivity was precipitated in this step, demonstrating complete removal of free amino acids. The dried pellet was redissolved and processed for scintillation counting (data of Table 1 and Fig. 4b) or processed as follows for amino acid analysis (Fig. 2b): the pellet was dissolved in 600 □l 50 mM $NH_4HCO_3$, 0.5% SDS, 3 mM $CaCl_2$ containing 0.25 mg pronase (Roche) followed by incubation at 37°C for 20 h. The sample was lyophilized, dissolved in 600 μl of 7 N HCl containing 4 mg of glutamic acid and 6 mg of photo-Met and heated to 70°C for 24 h. A Dowex 50WX8 column (1.6 x 24 cm) was prepared[18] and equilibrated with 0.1 M sodium citrate pH 3.8. The sample was concentrated to 50 μl in a speed-vak, diluted to 1 ml with 0.1 M sodium citrate pH 3.8, and loaded onto the column. The column was eluted at 24°C at a flow rate of 20 ml/h with 70 ml 0.1 M sodium citrate pH 3.8, 26 ml 0.1 M sodium citrate pH 4.3 and 100 ml of 0.1 M sodium citrate pH 4.5. Fractions of 3 ml were collected and assayed for absorbance at 350 nm, radioactivity by scintillation counting, and amino groups by the fluorescamine method[44].

**Example 2**

Incorporation of amino acids of the invention into proteins

To demonstrate biosynthetic incorporation of photo-Met into proteins, mammalian fibroblast-like (COS7) cells were cultivated in a growth medium supplemented with [1-$^{14}$C]photo-Met. To decrease competition for activation by tRNA synthetases, media devoid of the structurally related amino acids Leu and Met (LM-2) or, for some experiments, devoid of Leu, Met, Ile and Val (LM-4) were used. Proteins incorporated radioactivity as shown by autoradiography after separation by SDS-PAGE (Fig. 2a, lane 3). To prove that the radioactivity represents intact [1-$^{14}$C]photo-Met, labeled proteins were subjected to total hydrolysis, followed by separation of amino acids on a polystyrene ion exchange column[18]. 88 % of the radioactivity in the labeled proteins was intact [1-$^{14}$C]photo-Met (Fig. 2b, photo-Met). A small peak eluting slightly earlier than glutamic acid (Fig 2 B, Glu) contained 7 % of the radioactivity. This peak was also observed if free [1-$^{14}$C]photo-Met was heated with HCl under the conditions of the hydrolysis (data not shown) and therefore represents a product of thermal decomposition of the amino acid. Under the conditions employed, radioactivity originating from degradation/re-integration would be expected to appear as aspartate and glutamate (oxidative release of [$^{14}$C]$CO_2$, metabolism via pyruvate carboxylase, citric acid cycle and transamination to Gln and Asn that will be hydrolyzed to Glu and Asp by hot HCl). However, no radioactivity was incorporated via this pathway. We conclude that at least 95 % of incorporated radioactivity represents intact [1-$^{14}$C]photo-Met. To obtain quantitative data on the frequency of incorporation of photo-Met into newly synthesized protein, we performed co-labeling with [1-$^{14}$C]photo-Met and [$^{3}$H]Phe. Comparison of the amount of incorporated isotopes (Table 1) showed that the frequency of incorporation of [1-$^{14}$C]photo-Met was 14 % of that of phenylalanine, corresponding to 0.7% of total amino acids. Two in three hundred amino acids in newly synthesized protein will be photo-Met.

Table 1: Determination of the frequency of incorporation of photo-Met into newly synthesized protein. COS7 cells were grown for 22 h in LM-2 in the presence of [$^{3}$H]Phe and [1-$^{14}$C]photo-Met. Protein-bound radioactivity was determined by scintillation counting of protein pellets after chloroform/methanol precipitation. Data are average $\pm$ SD of triplicate determinations.

| | [$^{3}$H]Phe | [1-$^{14}$C]photo-Met |
|---|---|---|
| Total added (nCi/dish) | 1,000 | 5,000 |
| Specific activity (nCi/nmol) | 4.2 | 4.7 |
| Protein-bound activity (nCi/dish) | 14.5 $\pm$ 1.4 | 2.34 $\pm$ 0.23 |
| Incorporated amino acid (nmol/ dish) | 3.45 $\pm$ 0.34 | 0.50 $\pm$ 0.05 |
| Relative frequency | 4.8 %[45] | 0.70 % |

The amino acids of the invention compete with their naturally occurring counterparts for incorporation

The structures of the three photo-amino acids shown in Fig. 1 a suggest that photo-Ile, photo-Leu and photo-Met might replace Ile, Leu and Met, respectively. If so, addition of the complementary naturally occurring amino acid should strongly reduce incorporation and inhibit photo-crosslinking. We transfected cells with the membrane protein PGRMC1[24] and competed cross-linking by both photo-Leu and photo-Met with Met, Val, Leu and Ile. cross-linking by photo-Leu (Fig. 4a, lanes 3-7) was competed mainly by Leu, cross-linking by photo-Met (Fig. 4b, lanes 3-7) mainly by Met, and to a lower extent by Leu. Val and Ile did not compete, but even enhance, cross-linking by photo-Met. To find out if competition for cross-linking reflects competition for incorporation, we competed incorporation of [1-$^{14}$C]photo-Met with the same set of amino acids (Fig. 4c). Consistent with their effect on cross-linking efficiency, Met and Leu strongly competed for incorporation of [1-$^{14}$C]photo-Met, while Val and Ile slightly stimulated incorporation. It should be noted that Met, Val, Leu and Ile all are transported into cells by the L-type transport system[25], which presumably also transports the three photo-amino acids. The strong difference in competition by Met and Leu versus Val and Ile excludes competition at the level of uptake.

**Example 3**

Non-toxicity of the amino acids of the invention

Unnatural amino acids frequently are toxic to cells. To find out if this is the case for photo-amino acids of the invention, we monitored in COS7 cells protein biosynthesis by incorporation of [$^{3}$H]Phe and viability by trypan blue

exclusion of cells grown in the presence or absence of the photo-amino acids photo-Leu, photo-Met and photo-Ile. The results are summarized in Table 2. Due to the lack of two essential amino acids in the growth medium, protein biosynthesis is reduced to about 65% of that in a full growth medium. Addition of either photo-Leu or photo-Ile had no significant effect on protein biosynthesis, while addition of photo-Met increased protein biosynthesis. Although cells grew slightly slower than in full growth medium, viability (Table 2) and cell morphology (not shown) were unaffected. Also, photoactivation by UV light did not affect viability of the cells. Irradiation for three minutes, sufficient to activate more than 99% of the activatable groups, resulted in no increase of trypan blue positive cells (see Table 3).

Table 2: Effects of azi-amino acids on cellular protein synthesis and cell viability. Cells were grown in LM-2 with addition of a constant trace amount of [$^3$H]Phe and of non-radioactive amino acids as indicated. Protein-bound radioactivity was determined by scintillation counting of protein pellets after chloroform/methanol precipitation. The value obtained for growth in full DMEM medium is set to 100. Data are average ± SD of triplicate determinations. Cell viability was determined by trypan blue exclusion and is expressed as percent ± SD of viable cells (n=6).

| LM-2 Medium supplement | none | photo-Leu | photo-Met | photo-Ile | photo-Leu, photo-Met |
|---|---|---|---|---|---|
| Protein synthesis | 65.2 ± 8.7 | 61.5 ± 1.7 | 76.0 ± 6.4 | 62.8 ± 2.8 | 62.5 ± 3.7 |
| Cell viability | 99.7 ± 0.2 | 99.8 ± 0.2 | 99.8 ± 0.1 | 99.6 ± 0.3 | 99.7 ± 0.1 |

Table 3: Effects of UV irradiation in the absence or presence of photo-amino acids on cell viability. Cells were grown in LM-2 with addition of photo-amino acids as indicated. Cells were washed twice in PBS and irradiated for the times indicated. Cell viability was determined by trypan blue exclusion either directly after irradiation or after a one-hour chase period during which the cells were kept at 37 degree Celsius in PBS. Data are expressed as percent ± SD of viable cells (for each condition, six independent fields of 100-150 cells each were counted).

| | LM-2 | LM-2 + photo-Leu | LM-2 + photo-Met |
|---|---|---|---|
| no UV | 99.8±0.2 | 99.5±0.3 | 99.8±0.3 |
| no UV + 1 h chase | 99.3 ± 0.3 | 99.3 ± 0.5 | 99.4 ± 0.4 |
| 1 min UV | 99.4 ± 0.4 | 99.5 ± 0.2 | 99.4 ± 0.5 |
| 1 min UV + 1 h chase | 94.6 ± 1.3 | 95.5 ± 3.0 | 96.1 ± 1.3 |
| 3 min UV | 99.5 ± 0.2 | 99.6 ± 0.2 | 99.5 ± 0.4 |
| 3 min UV + 1 h chase | 94.8 ± 0.9 | 95.3 ± 1.8 | 91.9 ± 6.8 |
| 10min UV | 99.0 ± 0.3 | 99.5 ± 0.3 | 99.4 ± 0.3 |
| 10 min UV + 1 h chase | 94.7 ± 1.8 | 91.5 ± 4.0 | 91.9 ± 1.4 |
| 30 min UV | 98.7 ± 0.5 | 98.8 ± 0.4 | 98.7 ± 0.4 |
| 30 min UV + 1 h chase | 15.2 ± 3.0 | 17.9 ± 3.2 | 17.4 ± 5.3 |

**Example 4**

Proteins comprising amino acids of the invention are functional

We furthermore tested, whether proteins produced by cells grown with photo-amino acids are functional or not. For that, we transfected cells with GFP-fusion constructs of the small GTPase Rab5 and of mutated Rab5 that is constitutively activated (GFP-Rab5QL) or dominant negative (GFP-Rab5SN)[19] and monitored the GFP fluorescence by confocal microscopy. Four hours after transfection, when cells were switched to media containing photo-amino acids, no GFP signal was detectable (see Figure 7). After 24 h, GFP-Rab5 was found in a punctate pattern representing endosomes, GFP-Rab5QL localized to enlarged endosomes and GFP-Rab5SN displayed a cytosolic staining, all consistent with previous reports[19]. Both, staining intensity and localization were unaffected by the presence of either of the photo-amino acids. Taking into account a frequency of substitution of photo-Met for Met of 35% (see below) and the amounts of Met in Rab5 and GFP, more than 80% of the fusion protein will have photo-Met in both the Rab5- and the GFP-part of the fusion, more than 99% in at least one of the two parts. We conclude that photo-amino acids do not impair the function of Rab5 or of GFP. To corroborate this with a quantitative assay, we measured the activity of beta-galactosidase that was expressed in the absence or presence of photo-amino acids (see Table 4). Activity in LM-2 labeling medium was 79% of that in full DMEM. The addition of either of the three photo-amino acids or of a mixture of

photo-Met and photo-Leu had no significant additional effect. E. coli beta-galactosidase contains 23 methionines of which on average 8 would be replaced by photo-Met; less than 0.01% of the protein are expected to have no substitution by photo-Met. We conclude that photo-amino acids are non-toxic to cultivated mammalian cells and can, at least partially, functionally replace branched chain amino acids and methionine.

Table 4: Activity of beta-galactosidase expressed in cells grown in the absence or presence of photo-amino acids. COS 7 cells were transfected with an expression vector for E. coli beta-galactosidase under the control of the human EF1-$\alpha$ promoter and kept in Optimem medium (with all essential amino acids) for four hours. After that media were replaced by DMEM + 10% FCS or LM-2 or LM-4 with various added photo-amino acids as indicated. After 20 h, cells were washed, lysed and assayed for beta-galactosidase activity using the Molecular Probes FluoReporter lacZ/ Galactosidase quantitation kit according to manufacturers instructions. Data are expressed as percent $\pm$ SD (n=6) of the value obtained in DMEM + FCS. Beta-galactosidase activity in untransfected cells was below 0.1 % of that of transfected cells.

| Medium | beta-galactosidase activity |
|---|---|
| DMEM + 10% FCS | 100 $\pm$ 11 |
| LM-4 | 60 $\pm$ 9 |
| LM-2 | 79 $\pm$ 5 |
| LM-2 + photo-Leu | 68 $\pm$ 9 |
| LM-2 + photo-Met | 73 $\pm$ 5 |
| LM-2 + photo-Ile | 93 $\pm$ 9 |
| LM-2 +photo-Leu +photo-Met | 68 $\pm$ 14 |

**Example 5**

**Cross-linking**

Cross-linking of a transfected protein

To study photocrosslinking induced by the photo-amino acids, we transfected COS7 cells with caveolin-1, a protein known to form dimers and higher oligomers upon chemical cross-linking in vitro[20], fed the cells with the three amino acids and looked for formation of covalent caveolin dimers upon UV irradiation (Fig. 3a). Controls showed no cross-linking in the absence of photo-amino acids (Fig. 3a, lane 1) and also no cross-linking in non-irradiated samples in the presence of photo-amino acids (Fig. 3a, lanes 2-4). However, occasionally weak cross-linking was induced also by irradiation in the absence of photo-amino acids (see an example in Fig. 4b, lane 1), consistent with previous reports on direct UV-crosslinking[21], In irradiated samples in the presence of photo-amino acids (Fig. 3a, lanes 5-7), significant formation of a cross-linked dimer was observed for photo-Leu and photo-Met, but very little cross-linking for photo-Ile.

Cross-linking of endogenous proteins

Crosslinking with the photo-amino acids is not confined to transfected proteins. Also the endogenous proteins stomatin[22] (Fig. 3b) or EEA1[23] (Fig. 3c) were cross-linked, both with efficiencies comparable to those of the chemical cross-linkers di-succinimidyl suberate (DSS) or para-formaldehyde. In particular for the large protein EEA1, cross-linking with photo-amino acids is superior to chemical cross-linking, since it allowed to resolve discrete cross-linked bands (Fig. 3c, lanes 1-3), while chemical cross-linking gave a high molecular weight aggregate that failed to resolve on SDS-PAGE (Fig. 3c, lanes 5,6).

Specificity of cross-linking

Crosslinking with the photo-amino acids should be very specific, due to the short half-live of the activated state carbene and the absence of any spacer molecule. To demonstrate this specificity, we analyzed cross-linking of subunits of the 19S proteasome regulatory particle. This complex contains 17 protein subunits. In contrast to the 20S core particle of the proteasome, no high-resolution structural data for the 19S complex are available. Interactions of individual subunits have been mapped by yeast two-hybrid analysis and several other in-vitro approaches resulting in a tentative map of interactions[26], [27]. We analyzed cytosol of photo-crosslinked HeLa cells with antibodies against two of the 19S subunits,

Rpt4(S10b) and Rpt1(S7) (Fig. 5). Rpt4 showed three distinct cross-linked bands of molecular weights of 120, 140 and 145 kDa (Fig. 5, lane 3), consistent with interactions with three other proteasome subunits, as concluded from previous in-vitro experiments[26]. In contrast, Rpt1 gave no prominent cross-linked band (Fig. 5, lane 4). A band at 115 kDa was already detected before irradiation (Fig. 5, lane 2) and is likely to represent cross-reactivity of the antibody. Only a weak band at 155 kDa appeared at much longer exposure time of the blot (not shown). Despite the high homology of the two proteins (44% identity, 62% similarity), no common interaction partners were detected, demonstrating the high specificity of the procedure. None of the cross-linked bands of Rpt4 contained Rpt1, consistent with the lack of interaction between these two subunits in previous studies[26], [27].

**Example 6**

Determination of interactions

The methods of the invention have been applied to the study of protein-protein interactions between membrane proteins. For that we selected a protein complex that is involved in the regulation of cellular lipid homeostasis. At least three membrane proteins, SCAP, Insig-1 and SREBP[28-30] are part of this protein assembly, and recent data suggest the existence of additional unidentified components[31]. The complex is located in the endoplasmatic reticulum where it responds to low cholesterol levels. PGRMC1, a progesterone-binding membrane protein of the endoplasmatic reticulum[24, 32], might be a further element involved in this process. In particular, we suspected it to be a direct interaction partner of Insig-1. In such a situation, when several components of a complex are known to interact with each other, co-immunoprecipitation and related pull-down techniques cannot conclusively demonstrate a direct interaction between two of the components, since any observed interaction may be mediated by a third partner. Therefore, we searched for a direct interaction by a covalent cross-link. PGRMC1-HA and Insig-1-myc were co-expressed in COS7 cells and photo-crosslinked using photo-Met. To detect the cross-linked proteins, detergent extracts were immunoprecipitated for PGRMC1 with anti-HA antibody and precipitates analyzed for Insig-1 by western blotting with anti-myc antibody. If cells were grown in the absence of photo-Met, only a small amount of co-immunoprecipitated Insig-1, but no cross-linked band was detected (Fig. 6a, lane 2). In cells grown with the cross-linking amino acid, a strong band appeared (Fig. 6a, lane 1) at the expected molecular weight of a Insig-1 x PGRMC1 cross-link. An identical band was detected using the inverted order of detection, i.e. immunoprecipitation with anti-myc followed by blotting with anti-HA antibody (Fig. 6a, lane 3). These results prove a direct interaction between the two proteins in living cells. By the same procedure, a direct interaction between SCAP and PGRMC1 was detected (Fig. 6b, lane 3), whereas no interaction between Insig-1 and an unrelated overexpressed protein with localization to the endoplasmic reticulum (Fig. 6b, lane 5,6) was found, confirming the specificity of the photo-crosslinking procedure.

**Example 7**

**Chemical synthesis**

This Example contains a detailed description of the chemical procedures and the characterization of products. The text is followed by a reaction scheme explaining the synthetic strategies used. Original 1 H- and 13C-NMR spectra (Figures 8 to 13) prove the identity and purity of final products and original UV-spectra (Figure 14) prove the presence of the intact diazirine ring in each of the final products.

The amino acids of the invention contain a photoactivatable diazirine ring, yielding a reactive carbene after light-induced loss of nitrogen. The three amino acids are designed to structurally resemble isoleucine, leucine and methionine. All three amino acids could be synthesized by $\alpha$-bromination[16] of the respective azi-carboxylic acids followed by aminolysis of the azi-bromo-carboxylic acids. While photo-Leu and photo-Met were obtained in gram-amounts, photo-Ile was obtained only in small amounts. In the case of photo-Met, Strecker-synthesis[17] starting from 4,4'-azi-pentanal also led to the desired product, but analogous attempts failed for photo-Leu and photo-Ile. Both synthetic pathways require drastic conditions, which are well tolerated by the diazirine rings. Enantiomers were resolved by enzymatic cleavage of N-acetyl derivatives to give pure L-photo-Leu, L-photo-Met and D-photo-Met (Fig. 1 b); no attempts were made to resolve the enantiomers and diastereomers of photo-Ile. $^{14}$C-labeled DL-photo-Met was obtained by Strecker-synthesis using [$^{14}$C]NaCN (Fig. 1b).

photo-Met

DL-2-Amino-5,5'-azi-hexanoic acid **(1)**

The compound was obtained by Strecker synthesis. Caution: Handling of NaCN is dangerous.

To a concentrated aqueous solution of 1.75 g of ammonium chloride were added 6 ml of conc. aqueous $NH_3$ and 1.61 g of NaCN. The mixture was cooled on ice and 3.45 g of 4,4'-azi-pentanal (Church & Weiss (1970)) were added dropwise under stirring, which was continued for 5 h at room temperature. Then, most of the water was evaporated under reduced pressure and, in a well-ventilated hood, 30 ml of concentrated HCl were added (Caution, possible release of highly toxic HCN gas). The mixture was stirred at 85°C for 6 h followed by evaporation of the water at reduced pressure. The dry residue was extracted with 40 ml hot methanol and the extract neutralized with N,N-dimethylethylamine. Upon standing for 2 days at -32°C a precipitate formed which was isolated and re-crystallized twice from 75% ethanol to yield 925 mg of pure **1**.

DL-2-Acetamino-5,5'-azi-hexanoic acid (**2**)

**1** (800 mg) was dissolved in 20 ml 1 N NaOH. Ice (15 g) was added and 1.43 ml acetic anhydride was added under stirring, which was continued for 5 min. The mixture was acidified to pH 2.5 with HCl and extracted with ethyl acetate. Evaporation of the solvent yielded 800 mg of pure **2**.
1H-NMR ($D_2O$): 0.91 ppm (s, 3H, 6-C$H_3$), 1.20 - 1.67 (m, 4H, 3,4-C$H_2$), 1.90 (s, 3H, acetyl-C$H_3$), 4.03 (dd, 1 H, 2-C*H*)
13C-NMR ($D_2O$): 18.42 ppm (6-C), 21.76 (acetyl-$CH_3$), 25.99 and 30.10 (3-C and 4-C), 26.02 (5-C), 54.49 (2-C), 173.48 and 178.65 (1-C and acetyl-CO)

L-2-amino-5,5'-azi-hexanoic acid **(3,** photo-Met)

**2** (1.5 g) was dissolved in 150 ml of water. The pH was adjusted to 7.5 by addition of $NH_4OH$, and 60 mg of porcine kidney acylase I (Sigma No. A-8376) were added. After stirring for 3 h at 30°C, the mixture was acidified with HCl, extracted with ether and filtered. The aqueous phase was loaded on a Dowex 50WX8 cation exchange resin ($H^+$-form, 30 ml). Flow-through and ether extract were kept for isolation of D-2-amino-5,5'-azi-hexanoic acid (see below). The column was washed with 10 bed volumes of water and eluted with 2 N $NH_4OH$. Product was obtained by lyophilization and crystallized from 70 % ethanol to yield 400 mg pure photo-Met (**3**).

UV: 1.9 mg/ml in water: 349.1 nm $\varepsilon$ = 55
in 50 mM HCl: 348.5 nm $\varepsilon$ = 55
in 150 mM NaOH: 350.7 nm $\varepsilon$ = 55

1H-NMR ($D_2O$): 1.04 ppm (s, 3H, 6-C$H_3$), 1.47 (m, 2H, 4-C$H_2$) and 1.77 (m, 2H, 3-C$H_2$), 3.70 (t, 1 H, 2-C*H*)
13C-NMR ($D_2O$): 18.76 ppm (6-C), 25.37 and 29.79 (3-C and 4-C), 26.45 (5-C), 54.44 (2-C), 174.47 (1-C)

D-2-amino-5,5'-azi-hexanoic acid (D-photo-Met)

The flow-through of the above cation exchange column was supplemented with 20 g of NaCl and extracted with 2 x 100 ml ethyl acetate. The extracts were combined with the above ether extract, dried and evaporated. The residual crude D-2-Acetamino-5,5'-azi-hexanoic acid was subjected to a second round of acylase digestion and separated from free L-amino acid to give 650 mg of D-2-Acetamino-5,5'-azi-hexanoic acid. The D-2-Acetamino-5,5'-azi-hexanoic acid was treated with 40 ml of 7 N HCl at 75°C for 16 h, followed by lyophilization and crystallization from 70 % ethanol to yield 300 mg pure product.

UV: 1.9 mg/ml in water: 349.5 nm $\varepsilon$ = 56
in 50 mM HCl: 348.3 nm $\varepsilon$ = 56
in 150 mM NaOH: 350.7 nm $\varepsilon$ = 56

DL-[1-$^{14}$C]-2-amino-5,5'-azi-hexanoic acid (**4**, [1-$^{14}$C]photo-Met)

A concentrated aqueous solution of 11 mg $NH_4Cl$ was mixed with 40 $\mu$l conc. $NH_4OH$, 10 mg NaCN and 1 mCi [$^{14}$C]NaCN (52 mCi/mmol, about 1 mg). 40 $\mu$l 4,4'-Azipentanal (Church & Weiss (1970)) were added and the mixture was shaken at 33°C for 16 h. Free ammonia and part of the water were removed in a speed-vac at reduced pressure. 200 $\mu$l of conc. HCl were added and the mixture was shaken at 85°C for 7 h. 1.2 ml of water were added, the mixture was shaken at 50°C for 10 min and centrifuged. The supernatant was loaded onto a Dowex 50W8 cation exchange column (2 ml). The column was washed with 10 ml of water and eluted with 2 N $NH_4OH$. Fractions containing radioactivity were pooled to yield 520 $\mu$Ci of raw **4.** UV-spectroscopy of the pooled fractions showed the intact diazirine ring (348 nm, $\varepsilon$ = 50). For further purification, the material was lyophilized and re-crystallized from 3 ml 70% ethanol to yield 250 $\mu$Ci (53 $\mu$mol, 7.3 mg) product of 96% radiochemical purity as determined by chiral TLC (Fig. 1, Macherey & Nagel

chiralplate, methanol/water/acetonitrile 1/1/4) or paper chromatography (Whatman 3 MM Chr, acetic acid/water/butanol 1/1/4, data not shown). In both assays, the substance behaved identical to authentic non-radioactive DL-2-amino-5,5'-azi-hexanoic acid 2.

photo-Leu

DL-2-Bromo-4,4'-azi-pentanoic acid (**5**)

The compound was obtained by bromination of 4,4'-azi-pentanoic acid according to the procedure described by Harrp et al (1975). Briefly, 4,4'-azi-pentanoic acid (Church & Weiss (1970)) (3 g, 23 mmol), $CCl_4$ (3 ml) and thionyl chloride (7 ml, 96 mmol) were heated to 65°C for 30 min. N-Bromosuccinimid (4.8 g, 27 mmol), $CCl_4$ (10 ml) and 0.1 ml of 48% HBr were added and the mixture was stirred at 55 °C for 4 h. The solvent and free bromine were removed under reduced pressure and the residue was extracted with 50 ml $CCl_4$. The solvent was removed and the crude product (2-Bromo-4,4'-azi-pentanoyl chloride) dissolved in acetone and hydrolyzed with aq $NaHCO_3$. The crude brominated free acid was obtained upon acidification with HCl and extraction with dichloromethane. The solvent was removed and the product filtered through silica gel in isohexane/ethyl acetate 9/1 followed by removal of the solvent.
UV 3.4 mg/ml in EtOH: 342.9 nm (ε = 48)
1 H-NMR ($CDCl_3$): 1.12 ppm (s, 3H, 5-C*H*$_3$), 2.07 (dd, 1 H, 3-C*H*$_2$), 2.25 (dd, 1 H, 3-C*H'*$_2$), 4.07 (t, 1 H, 2-C*H*), 10 (broad, 1 H, COO*H*)
13C-NMR ($CDCl_3$): 19.87 ppm (5-C), 24.09 (4-C), 38.23 (2-C), 39.81 (3-C), 174.36 (1-C)

DL-2-amino-4,4'-azi-pentanoic acid **(6)**

Aminolysis of **(5)** was performed in 100 ml ammonia-saturated methanol and 20 ml 25% aq ammonia for 5 days at 55°C. After evaporation of the ammonia, 20 ml of concentrated HCl were added followed by evaporation of the water at reduced pressure. The dry residue was extracted with 20 ml hot methanol and the extract neutralized with N, N-dimethylethylamine. Upon standing for 2 days at -32°C a precipitate formed which was isolated and re-crystallized twice from 70% ethanol to yield 450 mg pure **6**.

L-2-amino-4,4'-azi-pentanoic acid **(8,** photo-Leu)

**6** was acetylated as described above (see **2)** to obtain acetylation DL-2-acetamino-4,4'-azi-pentanoic acid **(7)** followed by enzymatic deacetylation as described above to give pure **8**.

UV: 1.9 mg/ml in water: 345.1 nm ε = 64
1.9 mg/ml in 50 mM HCl: 343.5 nm ε = 64
1.9 mg/ml in 150 mM NaOH: 348.7 nm ε = 64

1H-NMR ($D_2O$): 1.11 ppm (s, 3H, 5-C*H*$_3$), 1.74 (dd, J 8.2 Hz, 15.5 Hz, 1H, 3-C*H*$_2$), 2.07 (dd, J 5.5 Hz, 15.5 Hz, 1 H 3-C*H'*$_2$), 3.74 (dd, J 5.5 Hz, 8.2 Hz, 1 H, 2-C*H*)
13C-NMR ($D_2O$): 18.75 ppm (5-C), 24.05 (4-C), 36.43 (3-C), 51.38 (2-C), 174.03 (1-C)

photo-Ile

3-R,S-Methyl-4,4'-azi-pentanoic acid (**10**)

**10** was obtained from 3-R,S-Methyl-4-keto-pentanoic acid (**9**) by the procedure of Church and Weiss (1970).
UV: 2.3 mg/ml in EtOH: 346.2 nm ε=55
1 H-NMR ($CDCl_3$): 0.86 ppm (d, 3H, 3-methyl-CH3), 0.98 (s, 3H, 5-C*H*3), 1.93 (m, 1 H, 3-C*H*), 2.03 (dd, 1 H, 2-C*H*), 2.25 (dd, 1 H, 2-C*H'*), 11 ppm (broad, 1 H, COO*H*)
13C-NMR ($D_2O$): 14.93 ppm (3-methyl-C), 17.88 (5-C), 28.29 (4-C), 33.89 (3-C), 36.56 (2-C), 178.38 (1-C)

2R,S-Amino-3-R,S-methyl-4,4'-azi-pentanoic acid (**12**, photo-Ile)

Bromination (see **5**) of **10** gave 2R,S-Bromo-3-R,S-methyl-4,4'-azi-pentanoic acid (**11**), which was subjected to aminolysis with excess ammonia according to the procedure described above for photo-Leu, to give low yields of pure **12** (80 mg). No attempts were made to resolve the 2S,3R-form that would correspond to the configuration of natural isoleucin (2S,3S).

UV: 2.2 mg/ml in water: 344.6 nm ε=50
2.2 mg/ml in 50 mM HCl: 342.9 nm ε=50
2.2 mg/ml in 150 mM NaOH: 348.6 nm ε=50

1 H-NMR ($D_2O$): 0.82 ppm (d, 3H, 3-methyl-C$H_3$), 1.06 (s, 3H, 5-C$H_3$), 1.96 (m, 1 H, 3-C*H*), 3.65 (d, 1 H, 2-C*H*)
13C-NMR ($D_2O$): 12.03 ppm (3-methyl-C), 17.38 (5-C), 27.27 (4-C), 39.14 (3-C), 56.59 (2-C), 173.18 (1-C)

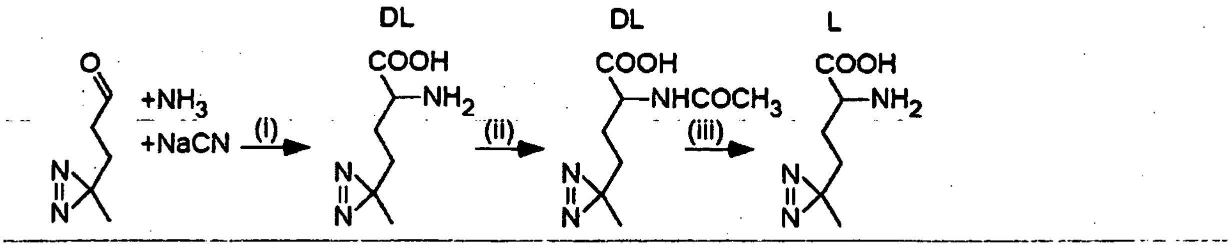


1 2 3, photo-Met

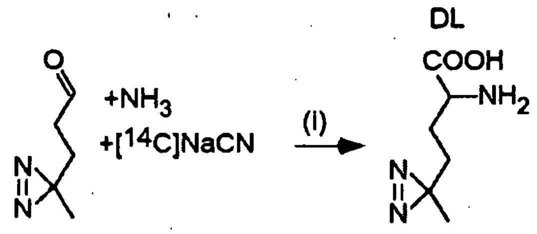


4, [1-14C]photo-Met

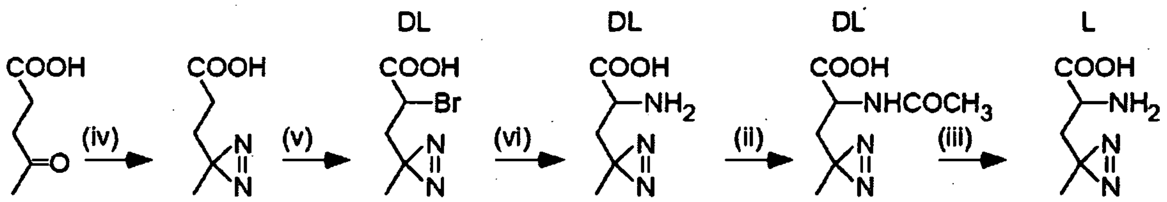


5 6 7 8, photo-Leu

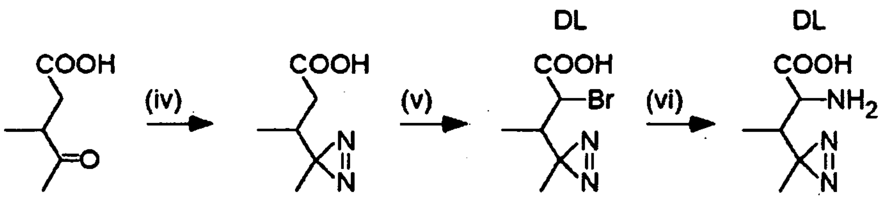


9 10 11 12, photo-Ile

**Reaction scheme**
(i) 5h at r.t., then conc HCl at 85°C for 6h. (ii) NaOH, acetic anhydride. (iii) kidney acylase 1
(iv) $NH_3$, $NH_2OSO_3H$, $I_2$. (v) $SOCl_2$. NBS + HBr, aq. $NaHCO_3$. (vi) conc. aq. $NH_3$, 55°C, 3-7 d

**References**

1. Ho, Y. et al. Systematic identification of protein complexes in Saccharomyces cerevisiae by mass spectrometry. Nature 415, 180-183 (2002).
2. Gavin, A.C. et al. Functional organization of the yeast proteome by systematic analysis of protein complexes. Nature 415, 141-147 (2002).
3. Gadek, T.R. Strategies and methods in the identification of antagonists of protein-protein interactions. Biotechniques Suppl, 21-24 (2003).
4. Ji, T.H. Bifunctional reagents. Methods Enzymol 91, 580-609 (1983).
5. Bayley, H. & Knowles, J.R. Photoaffinity labeling. Methods Enzymol. 46, 69-114 (1977).
6. Jacobs, S., Hazum, E., Shechter, Y. & Cuatrecasas, P. Insulin receptor: covalent labeling and identification of subunits. Proc Natl Acad Sci U S A 76, 4918-4921 (1979).
7. Thiele, C. & Fahrenholz, F. Photoaffinity labeling of central cholecystokinin receptors with high efficiency. Biochemistry 32, 2741-2746. (1993).
8. Kurzchalia, T.V. et al. The signal sequence of nascent preprolactin interacts with the 54K polypeptide of the signal recognition particle. Nature 320, 634-636 (1986).
9. Krieg, U.C., Walter, P. & Johnson, A.E. Photocrosslinking of the signal sequence of nascent preprolactin to the 54-kilodalton polypeptide of the signal recognition particle. Proc Natl Acad Sci U S A 83, 8604-8608 (1986).
10. Cornish, V.W. et al. Site-specific incorporation of biophysical probes into proteins. Proc Natl Acad Sci U S A 91, 2910-2915 (1994).
11. Chin, J.W., Martin, A.B., King, D.S., Wang, L. & Schultz, P.G. Addition of a photocrosslinking amino acid to the genetic code of Escherichiacoli. Proc Natl Acad Sci U S A 99, 11020-11024 (2002).
12. Deiters, A. et al. Adding amino acids with novel reactivity to the genetic code of Saccharomyces cerevisiae. J Am Chem Soc 125, 11782-11783 (2003).
13. Sakamoto, K. et al. Site-specific incorporation of an unnatural amino acid into proteins in mammalian cells. Nucleic Acids Res 30, 4692-4699 (2002).
14. Monahan, S.L., Lester, H.A. & Dougherty, D.A. Site-specific incorporation of unnatural amino acids into receptors expressed in Mammalian cells. Chem Biol 10, 573-580 (2003).
15. Zhang, Z. et al. Selective incorporation of 5-hydroxytryptophan into proteins in mammalian cells. Proc Natl Acad Sci U S A 101, 8882-8887 (2004).
16. Harrp, D.N., Bao, L.Q., Black, C.J., Gleason, J.G. & Smith, R.A. An efficient alpha-halogenation of acyl chlorides by N-bromosuccinimide, N-chlorosuccinimide, and molecular iodine. J. Org. Chem. 40, 3420-3427 (1975).
17. Strecker, A. Ueber die kuenstliche Bildung der Milchsaeure und eines neuen, dem Glycocoll homologen Koerper. Liebigs Ann. Chem. 75, 27-45 (1850).
18. Moore, S. & Stein, W.H. Chromatography of amino acids on sulfonated polystyrene resins. J Biol Chem 192, 663-681 (1951).
19. Stenmark, H. et al. Inhibition of rab5 GTPase activity stimulates membrane fusion in endocytosis. Embo J 13, 1287-1296 (1994).
20. Sargiacomo, M. et al. Oligomeric structure of caveolin: implications for caveolae membrane organization. Proc Natl Acad Sci U S A 92, 9407-9411 (1995).
21. Thibonnier, M. The human platelet vasopressin receptor identification by direct ultraviolet photoaffinity labeling. J Biol Chem 262, 10960-10964 (1987).
22. Snyers, L., Umlauf, E. & Prohaska, R. Oligomeric nature of the integral membrane protein stomatin. J Biol Chem 273, 17221-17226 (1998).
23. Simonsen, A. et al. EEA1 links PI(3)K function to Rab5 regulation of endosome fusion. Nature 394, 494-498 (1998).
24. Falkenstein, E., Meyer, C., Eisen, C., Scriba, P.C. & Wehling, M. Full-length cDNA sequence of a progesterone membrane-binding protein from porcine vascular smooth muscle cells. Biochem Biophys Res Commun 229, 86-89 (1996).
25. Kanai, Y. et al. Expression cloning and characterization of a transporter for large neutral amino acids activated by the heavy chain of 4F2 antigen (CD98). J Biol Chem 273, 23629-23632 (1998).
26. Ferrell, K., Wilkinson, C.R., Dubiel, W. & Gordon, C. Regulatory subunit interactions of the 26S proteasome, a complex problem. Trends Biochem Sci 25, 83-88 (2000).
27. Fu, H., Reis, N., Lee, Y., Glickman, M.H. & Vierstra, R.D. Subunit interaction maps for the regulatory particle of the 26S proteasome and the COP9 signalosome. Embo J 20, 7096-7107 (2001).
28. Brown, M.S. & Goldstein, J.L. A proteolytic pathway that controls the cholesterol content of membranes, cells,

and blood. Proc Natl Acad Sci U S A 96, 11041-11048 (1999).
29. Yang, T. et al. Crucial step in cholesterol homeostasis: sterols promote binding of SCAP to INSIG-1, a membrane protein that facilitates retention of SREBPs in ER. Cell 110, 489-500 (2002).
30. Horton, J.D., Goldstein, J.L. & Brown, M.S. SREBPs: transcriptional mediators of lipid homeostasis. Cold Spring Harb Symp Quant Biol 67, 491-498 (2002).
31. Adams, C.M. et al. Cholesterol and 25-hydroxycholesterol inhibit activation of SREBPs by different mechanisms, both involving SCAP and insigs. J Biol Chem (2004).
32. Nolte, I., Jeckel, D., Wieland, F.T. & Sohn, K. Localization and topology of ratp28, a member of a novel family of putative steroid-binding proteins. Biochim Biophys Acta 1543, 123-130 (2000).
33. Thiele, C., Hannah, M.J., Fahrenholz, F. & Huttner, W.B. Cholesterol binds to synaptophysin and is required for biogenesis of synaptic vesicles. Nat Cell Biol 2, 42-49. (2000).
34. Hendrickson, T.L., de Crecy-Lagard, V. & Schimmel, P. Incorporation of nonnatural amino acids into proteins. Annu Rev Biochem 73, 147-176 (2004).
35. Kiick, K.L., Weberskirch, R. & Tirrell, D.A. Identification of an expanded set of translationally active methionine analogues in Escherichia coli. FEBS Lett 502, 25-30 (2001).
36. Link, A.J., Mock, M.L. & Tirrell, D.A. Non-canonical amino acids in protein engineering. Curr Opin Biotechnol 14, 603-609 (2003).
37. Rennert, O.M. & Anker, H.S. On the Incorporation of 5',5',5'-Trifluoroleucine into Proteins of E. Coli. Biochemistry 13, 471-476 (1963).
38. Doring, V. et al. Enlarging the amino acid set of Escherichia coli by infiltration of the valine coding pathway. Science 292, 501-504 (2001).
39. Mursinna, R.S. & Martinis, S.A. Rational design to block amino acid editing of a tRNA synthetase. J Am Chem Soc 124, 7286-7287 (2002).
40. Tang, Y. & Tirrell, D.A. Attenuation of the editing activity of the Escherichia coli leucyl-tRNA synthetase allows incorporation of novel amino acids into proteins in vivo. Biochemistry 41, 10635-10645 (2002).
41. Jones, S. & Thornton, J.M. Principles of protein-protein interactions. Proc Natl Acad Sci U S A 93, 13-20 (1996).
42. Dupree, P., Parton, R.G., Raposo, G., Kurzchalia, T.V. & Simons, K. Caveolae and sorting in the trans-Golgi network of epithelial cells. Embo J 12, 1597-1605. (1993).
43. Wessel, D. & Fluegge, U.I. A method for the quantitative recovery of protein in dilute solution in the presence of detergents and lipids. Anal. Biochem. 138, 141-143 (1984).
44. Udenfriend, S. et al. Fluorescamine: a reagent for assay of amino acids, peptides, proteins, and primary amines in the picomole range. Science 178, 871-872 (1972).
45. Tourasse, N.J. & Li, W.H. Selective constraints, amino acid composition, and the rate of protein evolution. Mol Biol Evol 17, 656-664 (2000).

**Further References**

Brunner, J. New photolabeling and crosslinking methods. Annu. Rev. Biochem. 62, 483-514 (1993).

Church, R.F.R. & Weiss, M.J. Diazirines. II. Synthesis and properties of small functionalized diazirine molecules. Some observations on the reaction of a diaziridine with the iodine-iodide ion system. J. Org. Chem. 35, 2465-2471 (1970).

Harrp, D.N., Bao, L.Q., Black, C.J., Gleason, J.G. & Smith, R.A. An efficient alpha-halogenation of acyl chlorides by N-bromosuccinimide, N-chlorosuccinimide, and molecular iodine. J. Org. Chem. 40, 3420-3427 (1975).

Hendrickson, T. L., Nomanbhoy, T. K., & Schimmel, P. Errors from selective disruption of the editing center in a tRNA synthetase. Biochemistry 39, 8180-8186 (2000).

Lee, J.W. & Lee, S.K. Mammalian two-hybrid assay for detecting protein-protein interactions in vivo. Methods Mol Biol 261, 327-336 (2004).

Miller, J. & Stagljar, I. Using the yeast two-hybrid system to identify interacting proteins. Methods Mol Biol 261, 247-262 (2004).

Nassal, M. 4'-(1-Azi-2,2,2-trifluoroethyl)phenylalanine, a photolabile carbene-generating analogue of phenylalanine.

J. Amer. Chem. Soc. 106, 7540-7545 (1984).

Santoro, S. W., Wang, L., Herberich, B., King, D. S., and Schultz, P. G. An efficient system for the evolution of aminoacyl-tRNA synthetase specificity. Nature Biotech. 20, 1044-1048 (2002).

Thaminy, S., Miller, J. & Stagljar, I. The split-ubiquitin membrane-based yeast two-hybrid system. Methods Mol Biol 261, 297-312 (2004).

Turro, N. J., Chan, Y., and Gould, I. R. Reactivity and intersystem crossing of singlet methylene in solution. J. Amer. Chem. Soc. 109, 2101-2107 (1987).

## Claims

**1.** An amino acid based on a naturally occurring α-amino acid, said α-amino acid being selected from the group consisting of Leu, Met, Ile and Val, wherein said amino acid differs from the naturally occurring counterpart in that the side chain contains a diazirine ring, and wherein

(a) in case of said naturally occurring counterpart being Met the carbon atom of the diazirine ring replaces the sulphur; and
(b) in case of said naturally occurring counterpart not being Met the carbon atom of the diazirine ring is the penultimate carbon atom of the side chain,

(i) wherein in case of said naturally occurring counterpart being Leu or Val one of the terminal methyl groups present in the side chain of said counterpart is absent in said amino acid.

**2.** The amino acid of claim 1, wherein the side chain is extended as compared to the naturally occurring counterpart by the insertion of one or more methylene groups such that the total number of carbon atoms does not exceed 8.

**3.** The amino acid of claim 2, wherein said one or more methylene groups are inserted adjacent to a methylene group present in said naturally occurring counterpart.

**4.** The amino acid of any one of claims 1 to 3, wherein one or more hydrogen atoms, said hydrogen atoms being attached to carbon atoms, are substituted with fluorine.

**5.** The amino acid of claim 4, wherein one or more methyl groups are replaced with trifluoromethyl.

**6.** The amino acid of any one of claims 1 to 5, wherein said amino acid has the following generic formula A:

$$H_2N\text{-}CH(COOH)\text{-}(CH_2)_n\text{-}C(N{=}N)\text{-}(CH_2)_m\text{-}CH_3 \qquad \text{Formula A}$$

wherein n is an integer from 1 to 3, m is an integer from 0 to 3, and the sum n+m is an integer from 1 to 4.

**7.** The amino acid of any one of claims 1 to 6, wherein said amino acid has one of the following formulae (I) to (III):

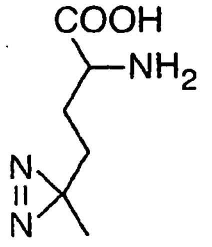

Formula (I)

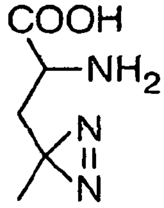

Formula (II)

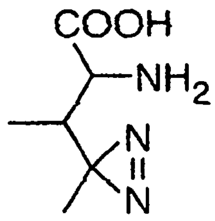

Formula (III)

**8.** The amino acid of any one of claims 1 to 7, wherein said amino acid is recognised and processed by a cognate wild-type aminoacyl-tRNA synthetase.

**9.** The amino acid of any one of claims 1 to 8, wherein said amino acid is processed by a wild-type ribosomal peptidyl transferase.

**10.** The amino acid of claim 8 or 9, wherein said aminoacyl-tRNA synthetase and/or said peptidyl transferase is of eukaryotic origin.

**11.** The amino acid of any one of claims 1 to 10, wherein said amino acid carries a protection group at the amino group and/or an activating group at the carboxy group.

**12.** The amino acid of any one of claims 1 to 11, wherein said amino acid is isotopically labelled.

**13.** The amino acid of claim 12, wherein the isotopic label is selected from the group consisting of 2H, 3H and $^{14}C$.

**14.** A method of synthesising a (poly)peptide with one or more amino acids according to any one of claims 1 to 13 incorporated, comprising

(a) allowing (poly)peptide synthesis to occur in an in vitro or cellular translation system in the presence one or more of said amino acids; and/or
(b) performing chemical synthesis of said (poly)peptide, wherein one or more of said amino acids are used as starting material, and wherein said amino acid(s) may be protected and/or activated.

**15.** The method of claim 14, wherein said translation system comprises wild-type aminoacyl-tRNA synthetase as the only source of aminoacyl-tRNA synthetase activity.

**16.** The method of claim 14 or 15, wherein said translation system comprises wild-type ribosomal peptidyl transferase as the only source of peptidyl transferase activity.

**17.** The method of claim 14 or 16, wherein said translation system comprises in addition or alternatively to wild-type aminoacyl-tRNA synthetase one or more modified aminoacyl-tRNA synthetases, wherein said modified aminoacyl-tRNA synthetase(s) is/are specific for said amino acid(s).

**18.** The method of claim 17, wherein one or more tRNAs with modified anticodon(s) are provided, and wherein said modified aminoacyl-tRNA synthetase(s) is/are capable of recognizing and processing said tRNA(s) with modified anticodon(s).

**19.** The method of claim 18, wherein said modified anticodon is complementary to the amber stop codon or complementary to a four-base codon.

**20.** The method of any one of claims 14 to 19, wherein said cellular translation system is comprised in a eukaryotic cell line.

**21.** The method of any one of claims 14 to 20, wherein the naturally occurring counterpart(s) of the amino acid(s) of the invention is/are absent.

**22.** The method of any one of claim 14 to 21, wherein said amino acid(s) is/are provided at a concentration of about 0.1 to about 10 mM.

**23.** A method for cross-linking interacting molecules in a biological system comprising a translation system, wherein at least one of said interacting molecules is a (poly)peptide, the method comprising the steps of:

(a) allowing (poly)peptide synthesis to occur in said biological system in the presence of one or more amino acids according to any one of claims 1 to 13; and
(b) irradiating said biological system such that the diazirine ring of said amino acid undergoes photolysis.

**24.** A method for cross-linking interacting molecules, wherein at least one of said interacting molecules is a (poly)peptide, the method comprising the steps of:

(a) performing chemical synthesis of said (poly)peptide, wherein one or more amino acids according to any one of claims 1 to 13 are used as starting material, and wherein said amino acid(s) may be protected and/or activated;
(b) bringing said (poly)peptide into contact with one or more test molecules; and
(c) irradiating the system obtained in step (b) such that the diazirine ring of said amino acid undergoes photolysis.

**25.** The method of claim 23 or 24, comprising the further step(s) of

(d) determining the identity of the interacting molecules; and/or
(e) determining the specific positions of said interacting molecules involved in the interaction of said interacting molecules.

**26.** The method of any one of claims 23 to 25, wherein said irradiating is effected with UV light of a wavelength between about 290 and about 380 nm.

**27.** The method of any one of claims 14 to 26, wherein at least one of the (poly)peptides is a membrane protein or membrane (poly)peptide.

**28.** The method of any one of claims 25 to 27, wherein a plurality of interactions is being determined, thereby providing one or more networks of biological interactions.

**29.** A kit comprising

(a) one or more amino acids according to any one of claims 1 to 13; and
(b) a manual with instructions for performing the method of any one of claims 14 to 28.

**30.** The kit of claim 29, furthermore comprising media devoid of one or more of the naturally occurring counterpart(s) of the amino acid(s) of any one of claims 1 to 13.

**31.** A method of synthesizing an amino acid of formula (I) as defined in claim 7, comprising the step of reacting 4,4'-azi-pentanal with $NH_3$, $NH_4Cl$ and NaCN.

**32.** A method of synthesizing an amino acid of formula (I) as defined in claim 7, comprising the steps of:

(a) reacting 5-oxo-hexanoic acid with $NH_3$, $NH_2OSO_3H$ and $I_2$;
(b) brominating the 5,5'-azi-hexanoic acid obtained in step (a); and
(c) aminolysis of the 2-bromo-5,5'-azi-hexanoic acid obtained in step (b).

**33.** A method of synthesizing an amino acid of formula (II) as defined in claim 7, comprising the steps of:

(a) reacting 4-oxo-pentanoic acid with $NH_3$, $NH_2OSO_3H$ and $I_2$;
(b) brominating the 4,4'-azi-pentanoic acid obtained in step (a); and
(c) aminolysis of the 2-brorno-4,4'-azi-pentanoic acid obtained in step (b).

**34.** A method of synthesizing an amino acid of formula (III) as defined in claim 7, comprising the steps of:

(a) reacting 3-methyl-4-oxo-pentanoic acid with $NH_3$, $NH_2OSO_3H$ and $I_2$;
(b) brominating the 3-methyl-4,4'-azi-pentanoic acid obtained in step (a); and
(c) aminolysis of the 2-bromo-3-methyl-4,4'-azi-pentanoic acid obtained in step (b).

**35.** The method of any one of claims 31 to 34 comprising the further step of separating the L-form of said amino acid from the D-form.

**Patentansprüche**

**1.** Aminosäure, basierend auf einer natürlich vorkommenden $\alpha$-Aminosäure, wobei die $\alpha$-Aminosäure ausgewählt ist aus der Gruppe bestehend aus Leu, Met, Ile und Val, wobei die Aminosäure sich vom natürlich vorkommenden Gegenstück dahingehend unterscheidet, dass die Seitenkette einen Diazirinring enthält, und wobei,

(a) das Kohlenstoffatom des Diazirinrings den Schwefel ersetzt, falls das natürlich vorkommende Gegenstück Met ist; und,
(b) das Kohlenstoffatom des Diazirinrings das vorletzte Kohlenstoffatom der Seitenkette ist, falls das natürlich vorkommende Gegenstück nicht Met ist,

(i) wobei eine der in der Seitenkette des Gegenstücks anwesenden terminalen Methylgruppen in der Aminosäure nicht vorhanden ist, falls das natürlich vorkommende Gegenstück Leu oder Val ist.

**2.** Aminosäure nach Anspruch 1, wobei die Seitenkette im Vergleich zum natürlich vorkommenden Gegenstück durch Einfügung einer oder mehrerer Methylengruppen verlängert ist, wobei die Gesamtzahl der Kohlenstoffatome 8 nicht übersteigt.

**3.** Aminosäure nach Anspruch 2, wobei die eine oder die mehreren Methylengruppen benachbart zu einer Methylengruppe eingefügt werden, die im natürlich vorkommenden Gegenstück anwesend ist.

**4.** Aminosäure nach einem der Ansprüche 1 bis 3, wobei ein oder mehrere Wasserstoffatome, die an Kohlenstoffatome gebunden sind, durch Fluor ersetzt sind.

**5.** Aminosäure nach Anspruch 4, wobei eine oder mehrere Methylgruppen durch Trifluoromethyl ersetzt sind.

**6.** Aminosäure nach einem der Ansprüche 1 bis 5, wobei die Aminosäure die folgende allgemeine Formel A hat:

$$H_2N\text{-}CH(COOH)\text{-}(CH_2)_n\text{-}C(N{=}N)\text{-}(CH_2)_m\text{-}CH_3 \qquad \text{Formel A}$$

wobei n eine ganze Zahl von 1 bis 3, m eine ganze Zahl von 0 bis 3, und die Summe n + m eine ganze Zahl von 1 bis 4 ist.

**7.** Aminosäure nach einem der Ansprüche 1 bis 6, wobei die Aminosäure eine der folgenden Formeln (I) bis (III) hat:

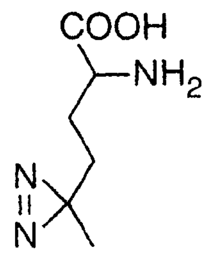

Formel (I)

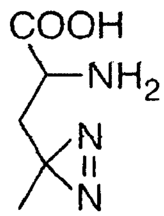

Formel (II)

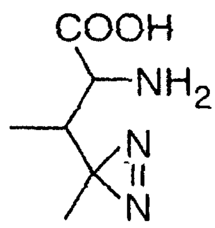

Formel (III)

8. Aminosäure nach einem der Ansprüche 1 bis 7, wobei die Aminosäure von einer zugehörigen Wildtyp-Aminoacyl-tRNA-Synthetase erkannt und prozessiert wird.

9. Aminosäure nach einem der Ansprüche 1 bis 8, wobei die Aminosäure von einer ribosomalen Wildtyp-Peptidyltransferase prozessiert wird.

10. Aminosäure nach Anspruch 8 oder 9, wobei die Aminoacyl-tRNA-Synthetase und/oder die Peptidyltransferase eukaryotischen Ursprungs ist/sind.

11. Aminosäure nach einem der Ansprüche 1 bis 10, wobei die Aminosäure eine Schutzgruppe an der Aminogruppe und/oder eine aktivierende Gruppe an der Carboxygruppe trägt.

12. Aminosäure nach einem der Ansprüche 1 bis 11, wobei die Aminosäure isotopenmarkiert ist.

13. Aminosäure nach Anspruch 12, wobei die Isotopenmarkierung ausgewählt ist aus der Gruppe bestehend aus $^{2}H$, 3H und $^{14}C$.

14. Verfahren zum Synthetisieren eines (Poly)Peptids, in das eine oder mehrere Aminosäuren nach einem der Ansprüche 1 bis 13 eingebaut sind, wobei das Verfahren umfasst

(a) Zulassen der (Poly)Peptidsynthese in einem in vitro oder zellulären Translationssystem in Gegenwart einer oder mehrerer der genannten Aminosäuren; und/oder
(b) Durchführen chemischer Synthese des (Poly)Peptids, wobei eine oder mehrere der genannten Aminosäuren als Ausgangsmaterial verwendet werden, und wobei die Aminosäure(n) geschützt und/oder aktiviert sein kann/können.

15. Verfahren nach Anspruch 14, wobei das Translationssystem Wildtyp-Aminoacyl-tRNA-Synthetase als die einzige Quelle von Aminoacyl-tRNA-Synthetaseaktivität umfasst.

16. Verfahren nach Anspruch 14 oder 15, wobei das Translationssystem ribosomale Wildtyp-Peptidyltransferase als die einzige Quelle von Peptidyltransferaseaktivität umfasst.

17. Verfahren nach Anspruch 14 oder 16, wobei das Translationssystem zusätzlich oder alternativ zu Wildtyp-Aminoacyl-tRNA-Synthetase eine oder mehrere modifizierte Aminoacyl-tRNA-Synthetasen umfasst, wobei die modifizierte(n) Aminoacyl-tRNA-Synthetase(n) spezifisch für die Aminosäure(n) ist/sind.

18. Verfahren nach Anspruch 17, wobei eine oder mehrere tRNAs mit modifiziertem/n Antikodon(s) bereitgestellt werden, und wobei die modifizierte(n) Aminoacyl-tRNA-Synthetase(n) in der Lage ist/sind, die tRNA(s) mit modifiziertem/n Antikodon(s) zu erkennen und zu prozessieren.

19. Verfahren nach Anspruch 18, wobei das modifizierte Antikodon zum Amber-Stopkodon oder einem vier-Basen Kodon komplementär ist.

20. Verfahren nach einem der Ansprüche 14 bis 19, wobei das zelluläre Translationssystem von einer eukaryotischen Zelllinie umfasst ist.

21. Verfahren nach einem der Ansprüche 14 bis 20, wobei das/die natürlich vorkommende(n) Gegenstück(e) der erfindungsgemäßen Aminosäure(n) abwesend ist/sind.

22. Verfahren nach einem der Ansprüche 14 bis 21, wobei die Aminosäure(n) in einer Konzentration von etwa 0,1 bis etwa 10 mM bereitgestellt ist/sind.

23. Verfahren zum Quervernetzen wechselwirkender Moleküle in einem biologischen System, das ein Translationssystem umfasst, wobei mindestens eines der interagierenden Moleküle ein (Poly)Peptid ist, wobei das Verfahren die folgenden Schritte umfasst:

(a) Zulassen der (Poly)Peptidsynthese im biologischen System in Gegenwart einer oder mehrerer Aminosäuren nach einem der Ansprüche 1 bis 13; und

(b) Bestrahlen des biologischen Systems, so dass der Diazirinring der Aminosäure Photolyse durchläuft.

**24.** Verfahren zum Quervernetzen wechselwirkender Moleküle, wobei mindestens eines der wechselwirkenden Moleküle ein (Poly)Peptid ist, wobei das Verfahren die folgenden Schritte umfasst:

(a) Durchführen der chemischen Synthese des (Poly)Peptids, wobei eine oder mehrere Aminosäuren nach einem der Ansprüche 1 bis 13 als Ausgangsmaterial verwendet werden, und wobei die Aminosäure(n) geschützt und/oder aktiviert sein kann/können;
(b) Inkontaktbringen des (Poly)Peptids mit einem oder mehreren Testmolekülen; und
(c) Bestrahlen des in Schritt (b) erhaltenen Systems, so dass der Diazirinring der Aminosäure Photolyse durchläuft.

**25.** Verfahren nach Anspruch 23 oder 24, umfassend den/die weiteren Schritt(e):

(d) Bestimmen der Identität der wechselwirkenden Moleküle; und/oder
(e) Bestimmen der spezifischen Positionen der wechselwirkenden Moleküle, die in die Wechselwirkung der wechselwirkenden Moleküle involviert sind.

**26.** Verfahren nach einem der Ansprüche 23 bis 25, wobei das Bestrahlen mit UV-Licht einer Wellenlänge zwischen etwa 290 und etwa 380 nm durchgeführt wird.

**27.** Verfahren nach einem der Ansprüche 14 bis 26, wobei mindestens eines der (Poly)Peptide ein Membranprotein oder Membran-(Poly)Peptid ist.

**28.** Verfahren nach einem der Ansprüche 25 bis 27, wobei eine Mehrzahl von Wechselwirkungen bestimmt wird, wodurch ein oder mehrere Netzwerke biologischer Wechselwirkungen bereitgestellt werden.

**29.** Kit umfassend

(a) eine oder mehrere Aminosäuren nach einem der Ansprüche 1 bis 13; und
(b) ein Handbuch mit Instruktionen zum Durchführen des Verfahrens nach einem der Ansprüche 14 bis 28.

**30.** Kit nach Anspruch 29, ferner umfassend Medien, die frei von einem oder mehreren der natürlich vorkommenden Gegenstück(en) der Aminosäure(n) nach einem der Ansprüche 1 bis 13 sind.

**31.** Verfahren zum Synthetisieren einer Aminosäure der Formel (I) wie in Anspruch 7 definiert, umfassend den Schritt des Umsetzens von 4,4'-Azi-Pentanal mit $NH_3$, $NH_4Cl$ und NaCN.

**32.** Verfahren zum Synthetisieren einer Aminosäure der Formel (I), wie in Anspruch 7 definiert, umfassend die Schritte:

(a) Umsetzen von 5-Oxo-Hexansäure mit $NH_3$, $NH_2OSO_3H$ und $I_2$;
(b) Bromieren der in Schritt (a) erhaltenen 5,5'-Azi-Hexansäure; und
(c) Aminolyse der in Schritt (b) erhaltenen 2-Brom-5-5'-Azi-Hexansäure.

**33.** Verfahren zum Synthetisieren einer Aminosäure der Formel (II), wie in Anspruch 7 definiert, umfassend die Schritte:

(a) Umsetzen von 4-Oxo-Pentansäure mit $NH_3$, $NH_2OSO_3H$ und $I_2$;
(b) Bromieren der in Schritt (a) erhaltenen 4,4'-Azi-Pentansäure; und
(c) Aminolyse der in Schritt (b) erhaltenen 2-Brom-4-4'-Azi-Pentansäure.

**34.** Verfahren zum Synthetisieren einer Aminosäure der Formel (III), wie in Anspruch 7 definiert, umfassend die Schritte:

(a) Umsetzen von 3-Methyl-4-Oxo-Pentansäure mit $NH_3$, $NH_2OSO_3H$ und $I_2$;
(b) Bromieren der in Schritt (a) erhaltenen 3-Methyl-4,4'-Azi-Pentansäure; und
(c) Aminolyse der in Schritt (b) erhaltenen 2-Brom-3-Methyl-4-4'-Azi-Pentansäure.

**35.** Verfahren nach einem der Ansprüche 31 bis 34, des weiteren umfassend den Schritt des Trennens der L-Form der Aminosäure von der D-Form.

## Revendications

1. Acide aminé basé sur un acide α-aminé existant à l'état naturel, ledit acide α-aminé étant choisi dans le groupe constitué de Leu, Met, Ile et Val, où ledit acide aminé diffère de la contrepartie existant à l'état naturel en ce que la chaîne latérale contient un cycle diazirine, et où

   (a) dans le cas où ladite contrepartie existant à l'état naturel est Met, l'atome de carbone du cycle diazirine remplace le soufre ; et
   (b) dans le cas où ladite contrepartie existant à l'état naturel n'est pas Met, l'atome de carbone du cycle diazirine est l'avant-dernier atome de carbone de la chaîne latérale,

      (i) où dans le cas où ladite contrepartie existant à l'état naturel est Leu ou Val, l'un des groupes méthyles terminaux présents dans la chaîne latérale de ladite contrepartie est absent dans ledit acide aminé.

2. Acide aminé selon la revendication 1, dans lequel la chaîne latérale est étendue par comparaison à la contrepartie existant à l'état naturel par l'insertion d'un ou plusieurs groupes méthylènes de telle manière que le nombre total d'atomes de carbone ne dépasse pas 8.

3. Acide aminé selon la revendication 2, dans lequel ledit ou lesdits groupes méthylènes est/sont inséré(s) adjacent (s) à un groupe méthylène présent dans ladite contrepartie existant à l'état naturel.

4. Acide aminé selon l'une quelconque des revendications 1 à 3, dans lequel un ou plusieurs atomes d'hydrogène, lesdits atomes d'hydrogène étant fixés à des atomes de carbone, sont substitués par du fluor.

5. Acide aminé selon la revendication 4, dans lequel un ou plusieurs groupes méthyles sont remplacés par un groupe trifluorométhyle.

6. Acide aminé selon l'une quelconque des revendications 1 à 5, où ledit acide aminé a la formule générique A suivante :

   $$H_2N\text{-}CH(COOH)\text{-}(CH_2)_n\text{-}C(N{=}N)\text{-}(CH_2)_m\text{-}CH_3 \qquad \text{Formule A}$$

   dans laquelle n est un nombre entier de 1 à 3, m est un nombre entier de 0 à 3, et la somme n + m est un nombre entier de 1 à 4.

7. Acide aminé selon l'une quelconque des revendications 1 à 6, où ledit acide aminé a l'une des formules (I) à (III) suivantes :

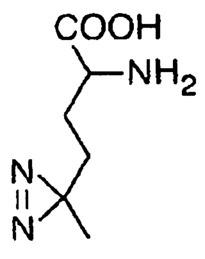
Formule (I)

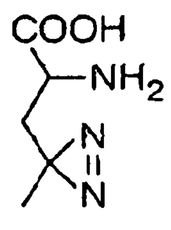
Formule (II)

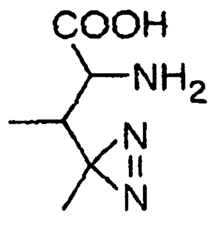
Formule (III)

8. Acide aminé selon l'une quelconque des revendications 1 à 7, où ledit acide aminé est reconnu et traité par une aminoacyl-ARNt synthétase de type sauvage apparentée.

9. Acide aminé selon l'une quelconque des revendications 1 à 8, où ledit acide aminé est traité par une peptidyl-transférase ribosomique de type sauvage.

**10.** Acide aminé selon la revendication 8 ou 9, où ladite aminoacyl-ARNt synthétase et/ou ladite peptidyl-transférase est d'origine eucaryote.

**11.** Acide aminé selon l'une quelconque des revendications 1 à 10, où ledit acide aminé porte un groupe protecteur au niveau du groupe amino et/ou un groupe activateur au niveau du groupe carboxy.

**12.** Acide aminé selon l'une quelconque des revendications 1 à 11, où ledit acide aminé est marqué par un isotope.

**13.** Acide aminé selon la revendication 12, où le marqueur isotopique est choisi dans le groupe constitué de $^{2}H$, $^{3}H$ et $^{14}C$.

**14.** Procédé de synthèse d'un (poly)peptide avec un ou plusieurs acides aminés selon l'une quelconque des revendications 1 à 13 incorporés, comprenant

(a) le fait de laisser la synthèse du (poly)peptide se produire dans un système de traduction *in vitro* ou cellulaire en présence d'un ou plusieurs desdits acides aminés ; et/ou
(b) la réalisation de la synthèse chimique dudit (poly)peptide, où un ou plusieurs desdits acides aminés sont utilisés comme matériau de départ, et où le(s)dit(s) acide(s) aminé(s) peut/peuvent être protégé(s) et/ou activé(s).

**15.** Procédé selon la revendication 14, dans lequel ledit système de traduction comprend l'aminoacyl-ARNt synthétase de type sauvage comme seule source d'activité aminoacyl-ARNt synthétase.

**16.** Procédé selon la revendication 14 ou 15, dans lequel ledit système de traduction comprend la peptidyl-transférase ribosomique de type sauvage comme seule source d'activité peptidyl-transférase.

**17.** Procédé selon la revendication 14 ou 16, dans lequel ledit système de traduction comprend en plus ou en variante de l'aminoacyl-ARNt synthétase de type sauvage une ou plusieurs aminoacyl-ARNt synthétases modifiées, où ladite/lesdites aminoacyl-ARNt synthétase(s) modifiée(s) est/sont spécifique(s) dudit/desdits acide(s) aminé(s).

**18.** Procédé selon la revendication 17, dans lequel un ou plusieurs ARNt avec un/des anticodon(s) modifié(s) sont fournis, et dans lequel ladite/lesdites aminoacyl-ARNt synthétase(s) modifiée(s) est/sont capable(s) de reconnaître et de traiter le(s)dits(s) ARNt avec un/des anticodon(s) modifié(s).

**19.** Procédé selon la revendication 18, dans lequel ledit anticodon modifié est complémentaire du codon d'arrêt ambre ou complémentaire d'un codon de quatre bases.

**20.** Procédé selon l'une quelconque des revendications 14 à 19, dans lequel ledit système de traduction cellulaire est compris dans une lignée cellulaire eucaryote.

**21.** Procédé selon l'une quelconque des revendications 14 à 20, dans lequel la/les contrepartie(s) existant à l'état naturel de l'acide/des acide(s) aminé(s) de l'invention est/sont absente(s).

**22.** Procédé selon l'une quelconque des revendications 14 à 21, dans lequel le(s)dit(s) acide(s) aminé(s) est/sont fourni(s) à une concentration d'environ 0,1 à environ 10 mM.

**23.** Procédé de réticulation de molécules interagissant dans un système biologique comprenant un système de traduction, où au moins une desdites molécules interagissant est un (poly)peptide, le procédé comprenant les étapes de :

(a) laisser la synthèse du (poly)peptide se produire dans ledit système biologique en présence d'un ou plusieurs acides aminés selon l'une quelconque des revendications 1 à 13 ; et
(b) irradier ledit système biologique de telle manière que le cycle diazirine dudit acide aminé subisse une photolyse.

**24.** Procédé de réticulation de molécules interagissant, dans lequel au moins une desdites molécules interagissant est un (poly)peptide, le procédé comprenant les étapes de :

(a) réaliser la synthèse chimique dudit (poly)peptide, où un ou plusieurs acides aminés selon l'une quelconque des revendications 1 à 13 sont utilisés en tant que matériau de départ, et où le(s)dit(s) acide(s) aminé(s) peut/peuvent être protégé(s) et/ou activé(s) ;
(b) mettre ledit (poly)peptide en contact avec une ou plusieurs molécules de test ; et
(c) irradier le système obtenu à l'étape (b) de telle manière que le cycle diazirine dudit acide aminé subisse une photolyse.

**25.** Procédé selon la revendication 23 ou 24, comprenant l'étape/les étapes supplémentaire(s) de :

(d) déterminer l'identité des molécules interagissant ; et/ou
(e) déterminer les positions spécifiques desdites molécules interagissant impliquées dans l'interaction desdites molécules interagissant.

**26.** Procédé selon l'une quelconque des revendications 23 à 25, dans lequel ladite irradiation est effectuée avec de la lumière UV à une longueur d'onde entre environ 290 et environ 380 nm.

**27.** Procédé selon l'une quelconque des revendications 14 à 26, dans lequel au moins un des (poly)peptides est une protéine membranaire ou un (poly)peptide membranaire.

**28.** Procédé selon l'une quelconque des revendications 25 à 27, dans lequel une pluralité d'interactions est déterminée, fournissant de cette manière un ou plusieurs réseaux d'interactions biologiques.

**29.** Kit comprenant

(a) un ou plusieurs acides aminés selon l'une quelconque des revendications 1 à 13 ; et
(b) un manuel avec des instructions pour réaliser le procédé selon l'une quelconque des revendications 14 à 28.

**30.** Kit selon la revendication 29, comprenant en outre un milieu privé d'une ou plusieurs des contreparties existant à l'état naturel de l'acide/des acide(s) aminé(s) selon l'une quelconque des revendications 1 à 13.

**31.** Procédé de synthèse d'un acide aminé de formule (I) telle que définie dans la revendication 7, comprenant l'étape consistant à faire réagir du 4,4'-azi-pentanal avec $NH_3$, $NH_4Cl$ et NaCN.

**32.** Procédé de synthèse d'un acide aminé de formule (I) telle que définie dans la revendication 7, comprenant les étapes de :

(a) faire réagir de l'acide 5-oxo-hexanoïque avec $NH_3$, $NH_2OSO_3H$ et $I_2$ ;
(b) bromer l'acide 5,5'-azi-hexanoïque obtenu à l'étape (a) ; et
(c) réaliser une aminolyse de l'acide 2-bromo-5,5'-azi-hexanoïque obtenu à l'étape (b).

**33.** Procédé de synthèse d'un acide aminé de formule (II) telle que définie dans la revendication 7, comprenant les étapes de :

(a) faire réagir de l'acide 4-oxo-pentanoïque avec $NH_3$, $NH_2OSO_3H$ et $I_2$ ;
(b) bromer l'acide 4,4'-azi-pentanoïque obtenu à l'étape (a) ; et
(c) réaliser une aminolyse de l'acide 2-bromo-4,4'-azi-pentanoïque obtenu à l'étape (b).

**34.** Procédé de synthèse d'un acide aminé de formule (III) telle que définie dans la revendication 7, comprenant les étapes de :

(a) faire réagir de l'acide 3-méthyl-4-oxo-pentanoïque avec $NH_3$, $NH_2OSO_3H$ et $I_2$ ;
(b) bromer l'acide 3-méthyl-4,4'-azi-pentanoïque obtenu à l'étape (a) ; et
(c) réaliser une aminolyse de l'acide 2-bromo-3-méthyl-4,4'-azi-pentanoïque obtenu à l'étape (b).

**35.** Procédé selon l'une quelconque des revendications 31 à 34, comprenant l'étape supplémentaire de séparer la forme L dudit acide aminé de la forme D.

## Figure 1

a

photo-Ile 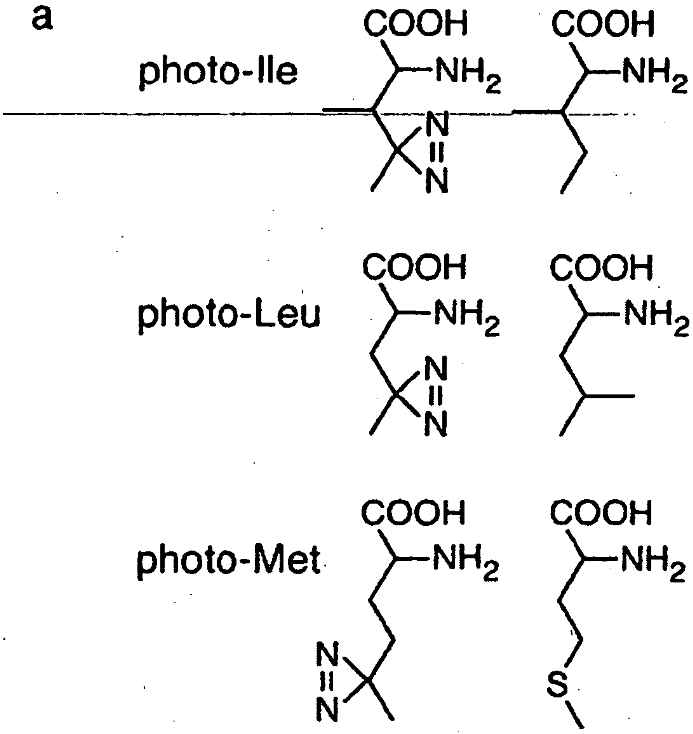 Ile

photo-Leu Leu

photo-Met Met

b

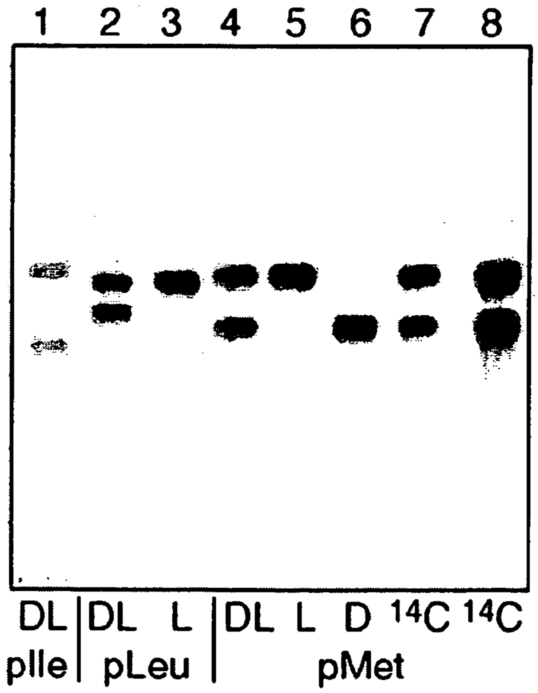

**Figure 2**

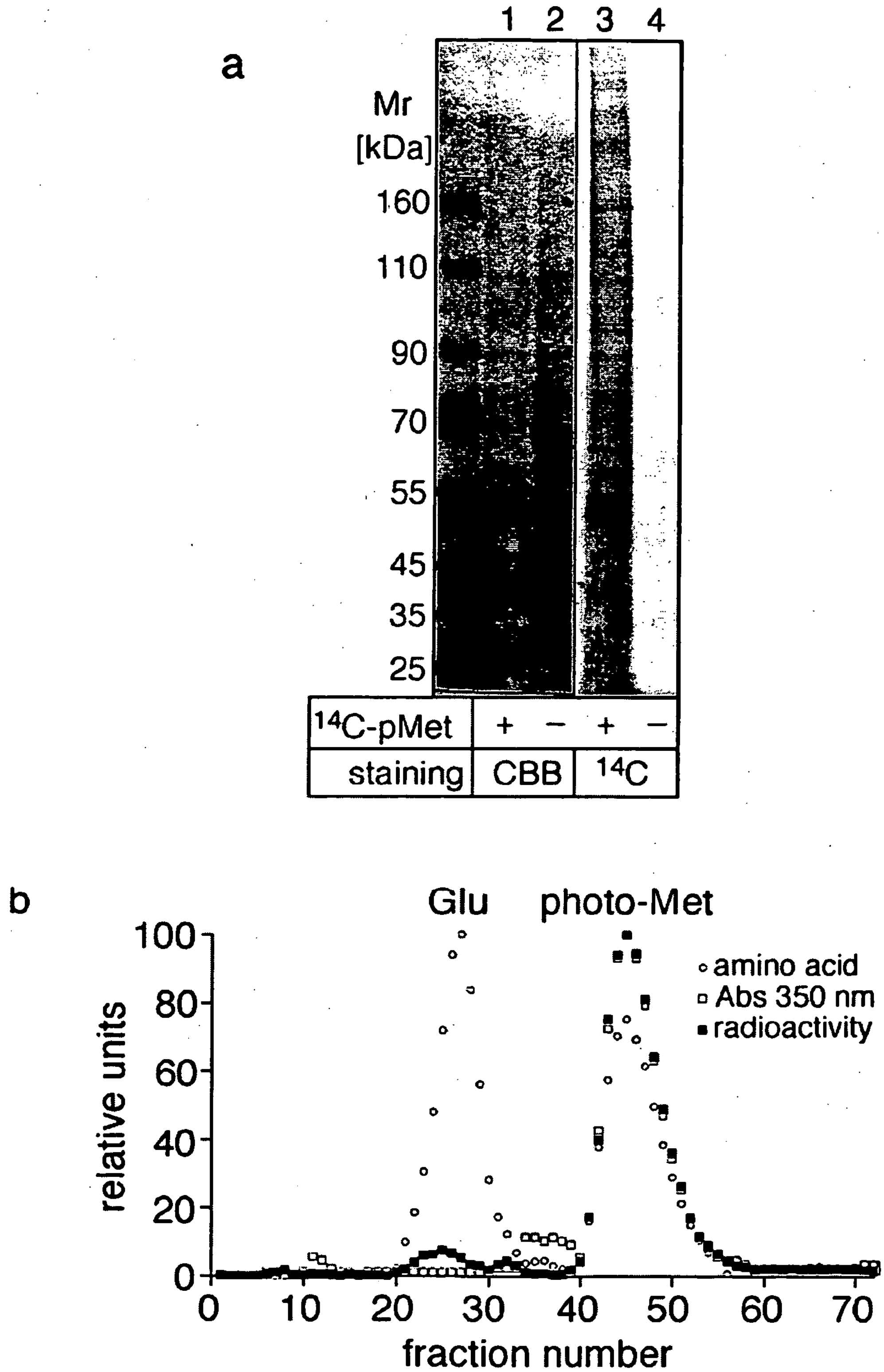
a
1 2 3 4
Mr
[kDa]
160
110
90
70
55
45
35
25
14C-pMet + − + −
staining CBB 14C
b
Glu
photo-Met
amino acid
Abs 350 nm
radioactivity
relative units
0
20
40
60
80
100
0 10 20 30 40 50 60 70
fraction number

## Figure 3

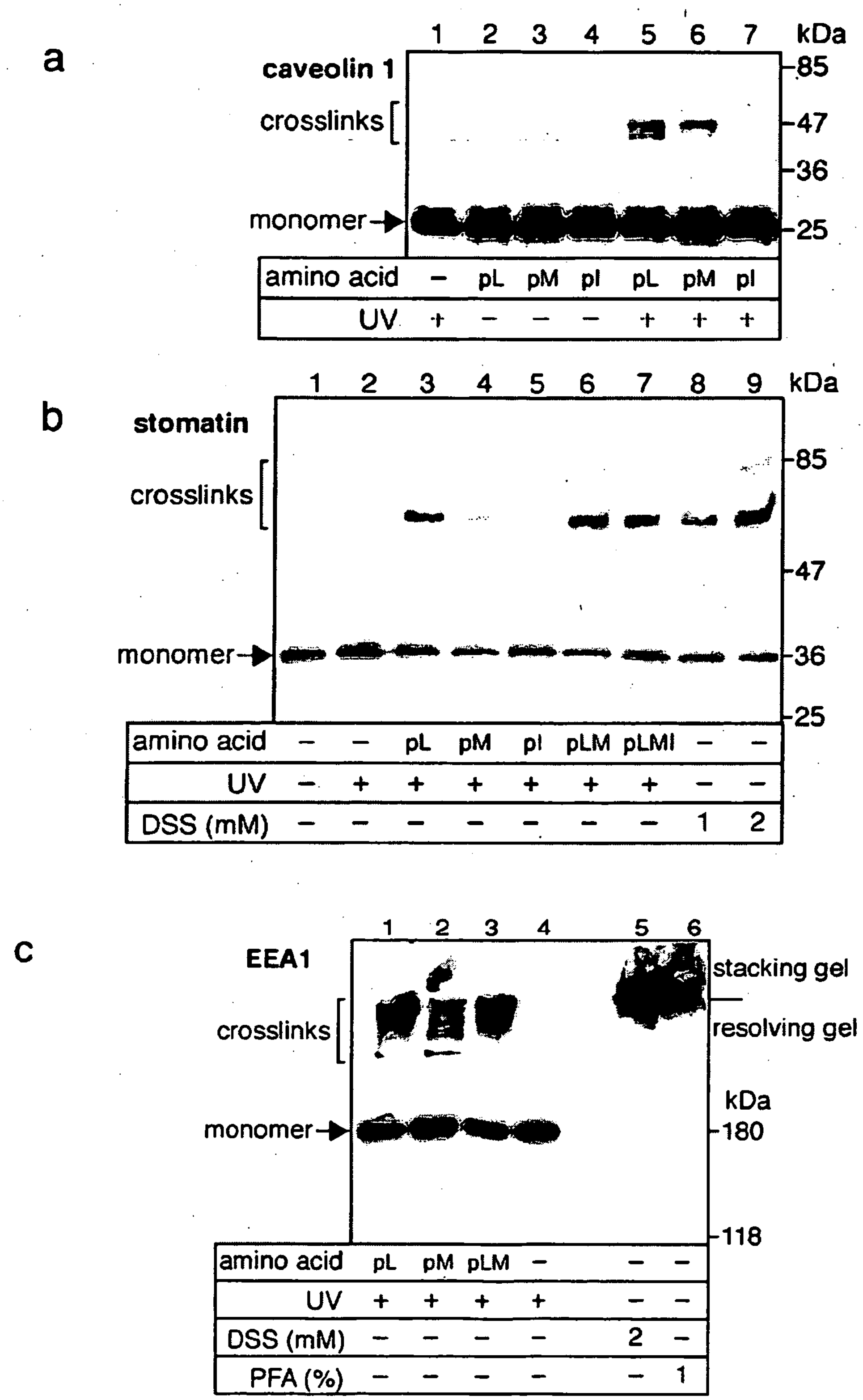
a
1 2 3 4 5 6 7 kDa
caveolin 1
crosslinks
monomer
85
47
36
25
amino acid – pL pM pI pL pM pI
UV + – – – + + +
b
1 2 3 4 5 6 7 8 9 kDa
stomatin
crosslinks
monomer
85
47
36
25
amino acid – – pL pM pI pLM pLMI – –
UV – + + + + + + – –
DSS (mM) – – – – – – – 1 2
c
1 2 3 4 5 6
EEA1
crosslinks
monomer
stacking gel
resolving gel
kDa
180
118
amino acid pL pM pLM – – –
UV + + + + – –
DSS (mM) – – – – 2 –
PFA (%) – – – – – 1

**Figure 4**

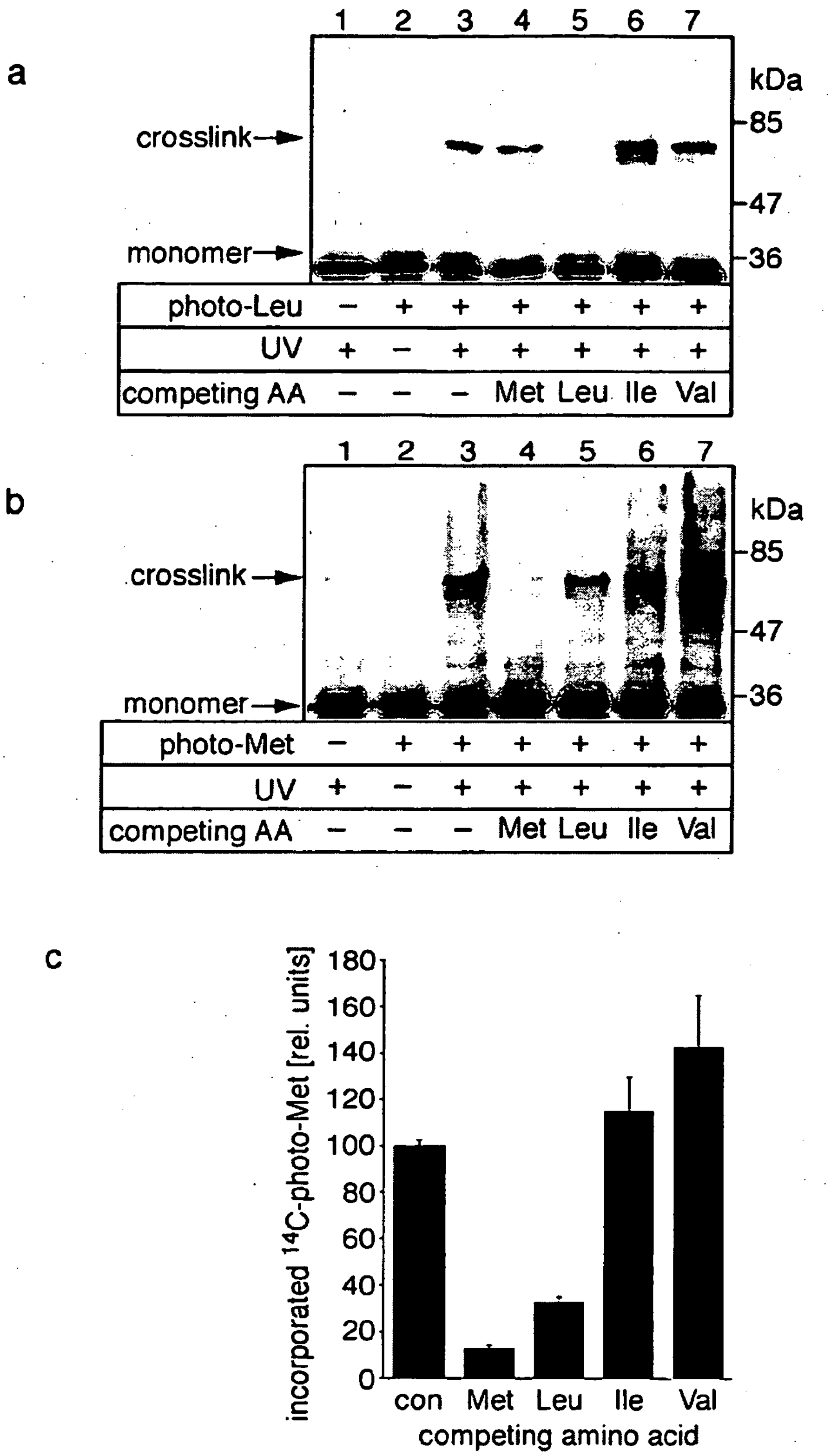
a
1 2 3 4 5 6 7
kDa
crosslink
monomer
85
47
36
photo-Leu – + + + + + +
UV + – + + + + +
competing AA – – – Met Leu Ile Val
b
1 2 3 4 5 6 7
kDa
crosslink
monomer
85
47
36
photo-Met – + + + + + +
UV + – + + + + +
competing AA – – – Met Leu Ile Val
c
incorporated 14C-photo-Met [rel. units]
180
160
140
120
100
80
60
40
20
0
con Met Leu Ile Val
competing amino acid

Figure 5

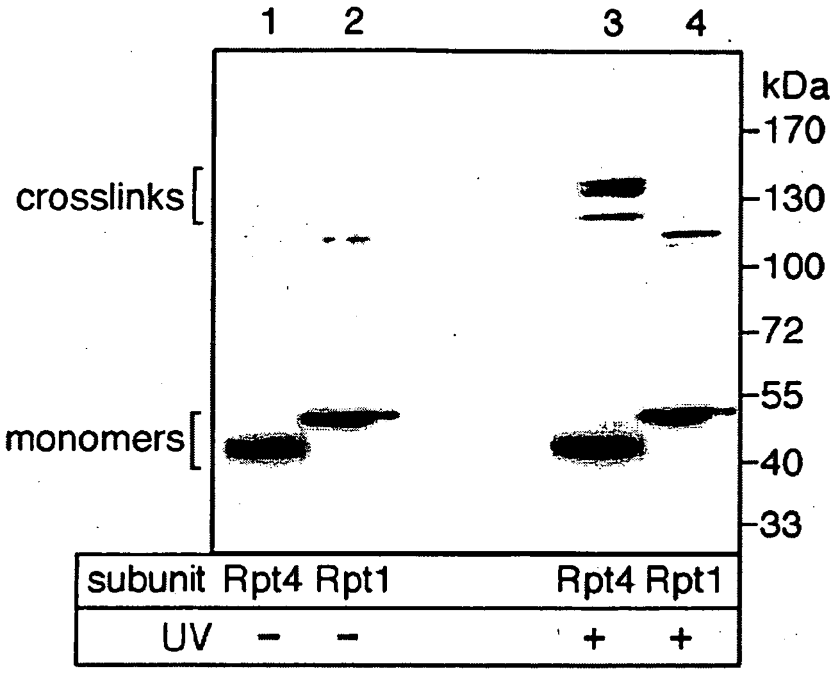
1 2 3 4
kDa
170
130
100
72
55
40
33
crosslinks
monomers
subunit Rpt4 Rpt1 Rpt4 Rpt1
UV – – + +

**Figure 6**

a

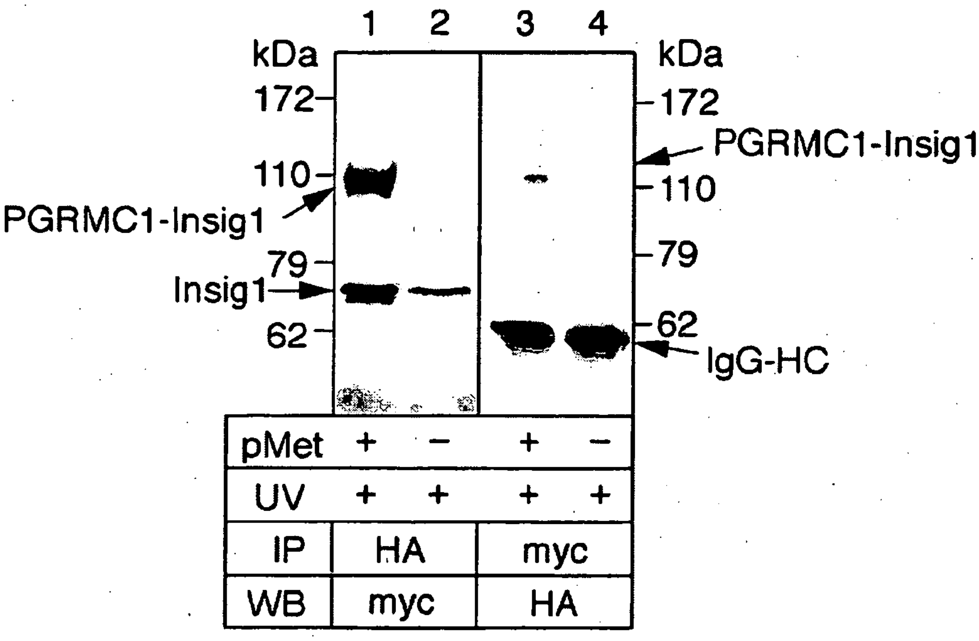
1 2 3 4
kDa
172
110
PGRMC1-Insig1
79
Insig1
62
kDa
172
PGRMC1-Insig1
110
79
62
IgG-HC
pMet + − + −
UV + + + +
IP HA myc
WB myc HA

b

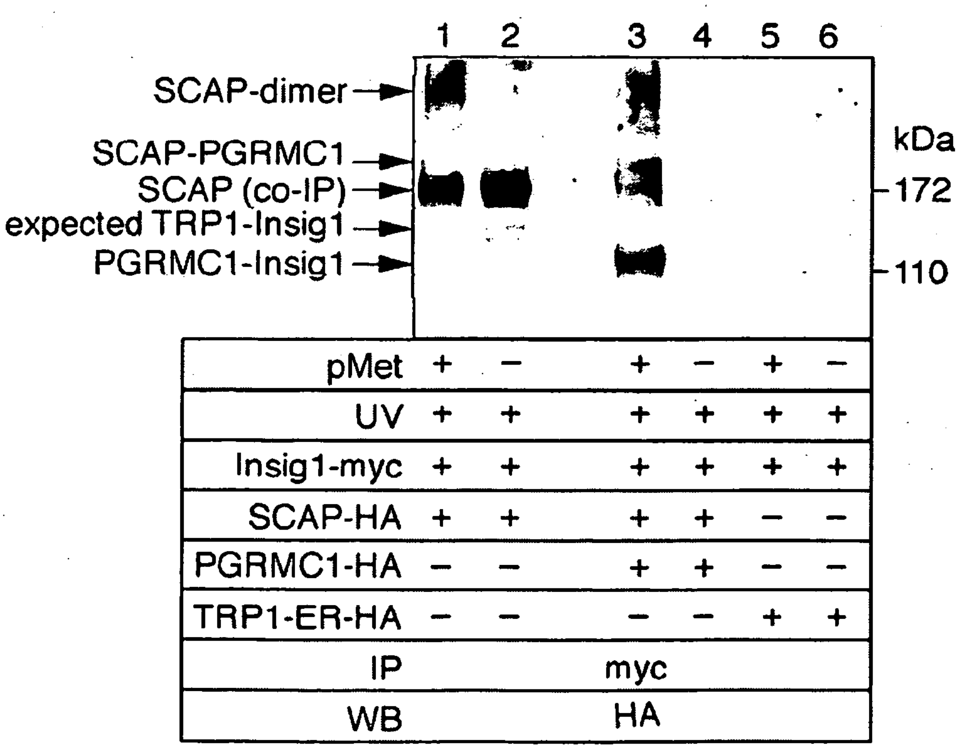
1 2 3 4 5 6
SCAP-dimer
SCAP-PGRMC1
SCAP (co-IP)
expected TRP1-Insig1
PGRMC1-Insig1
kDa
172
110
pMet + − + − + −
UV + + + + + +
Insig1-myc + + + + + +
SCAP-HA + + + + − −
PGRMC1-HA − − + + − −
TRP1-ER-HA − − − − + +
IP myc
WB HA

Figure 7

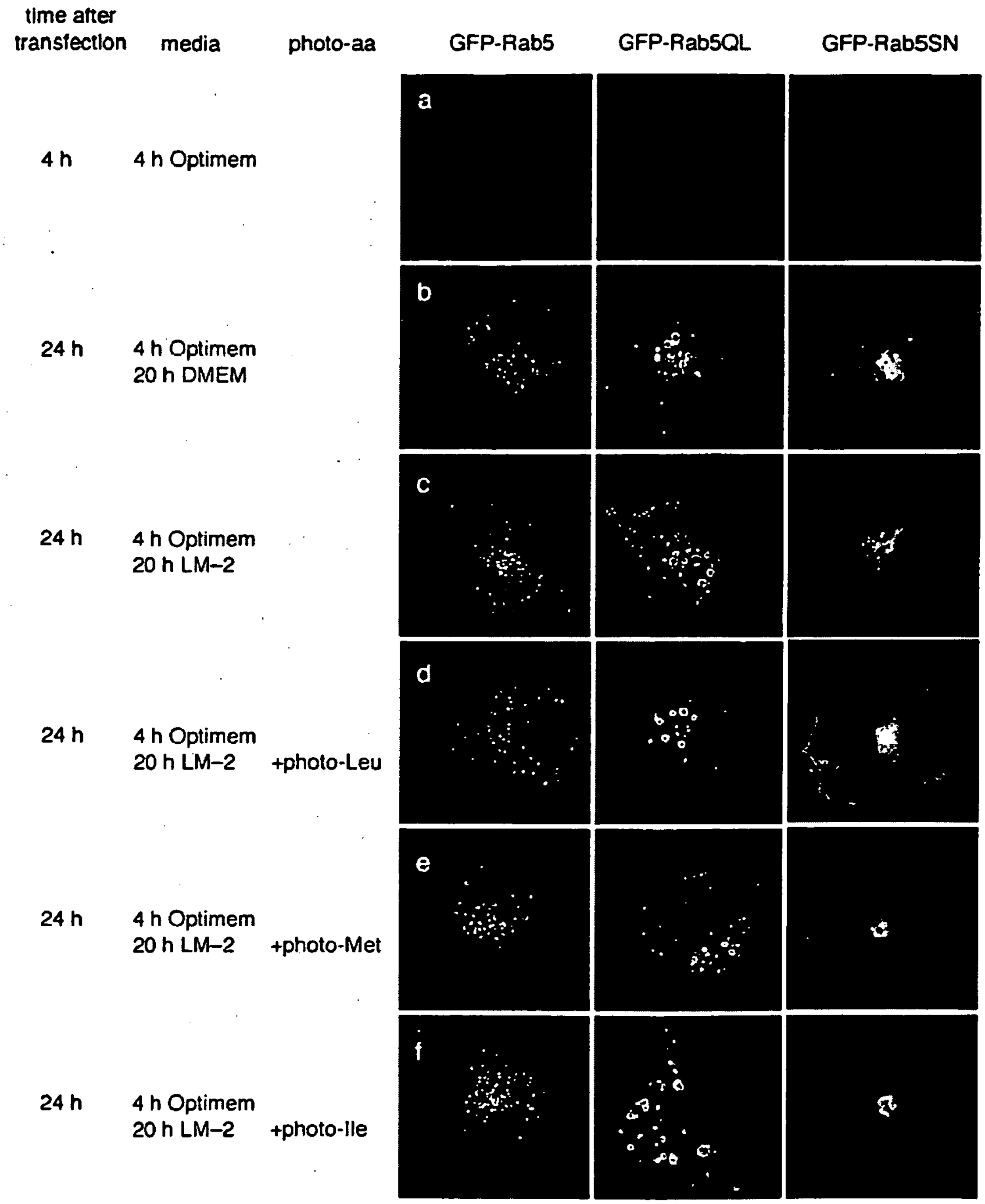
time after transfection
media
photo-aa
GFP-Rab5
GFP-Rab5QL
GFP-Rab5SN
a
4 h
4 h Optimem
b
24 h
4 h Optimem
20 h DMEM
c
24 h
4 h Optimem
20 h LM–2
d
24 h
4 h Optimem
20 h LM–2
+photo-Leu
e
24 h
4 h Optimem
20 h LM–2
+photo-Met
f
24 h
4 h Optimem
20 h LM–2
+photo-Ile

**Figure 8**

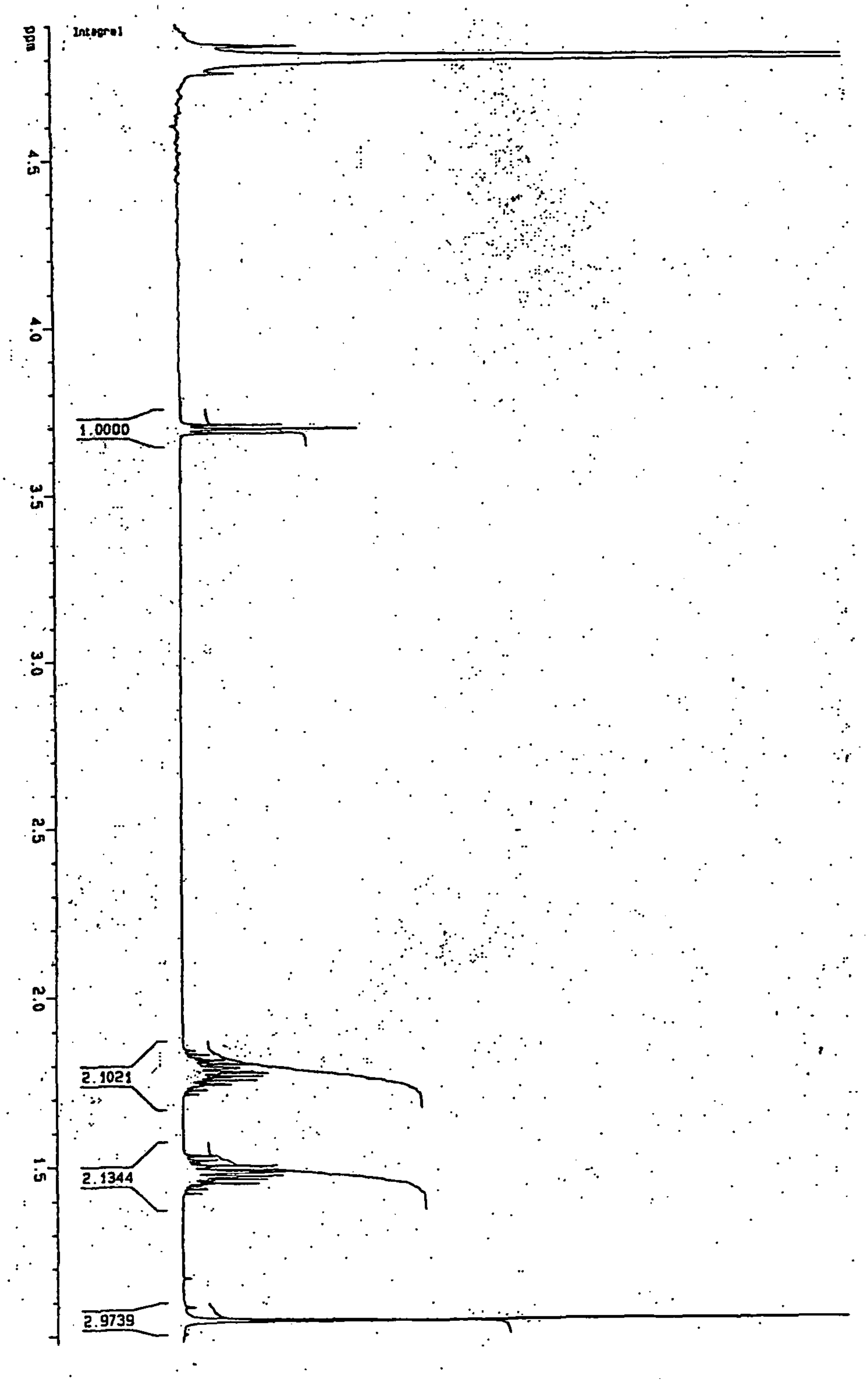

**Figure 9**

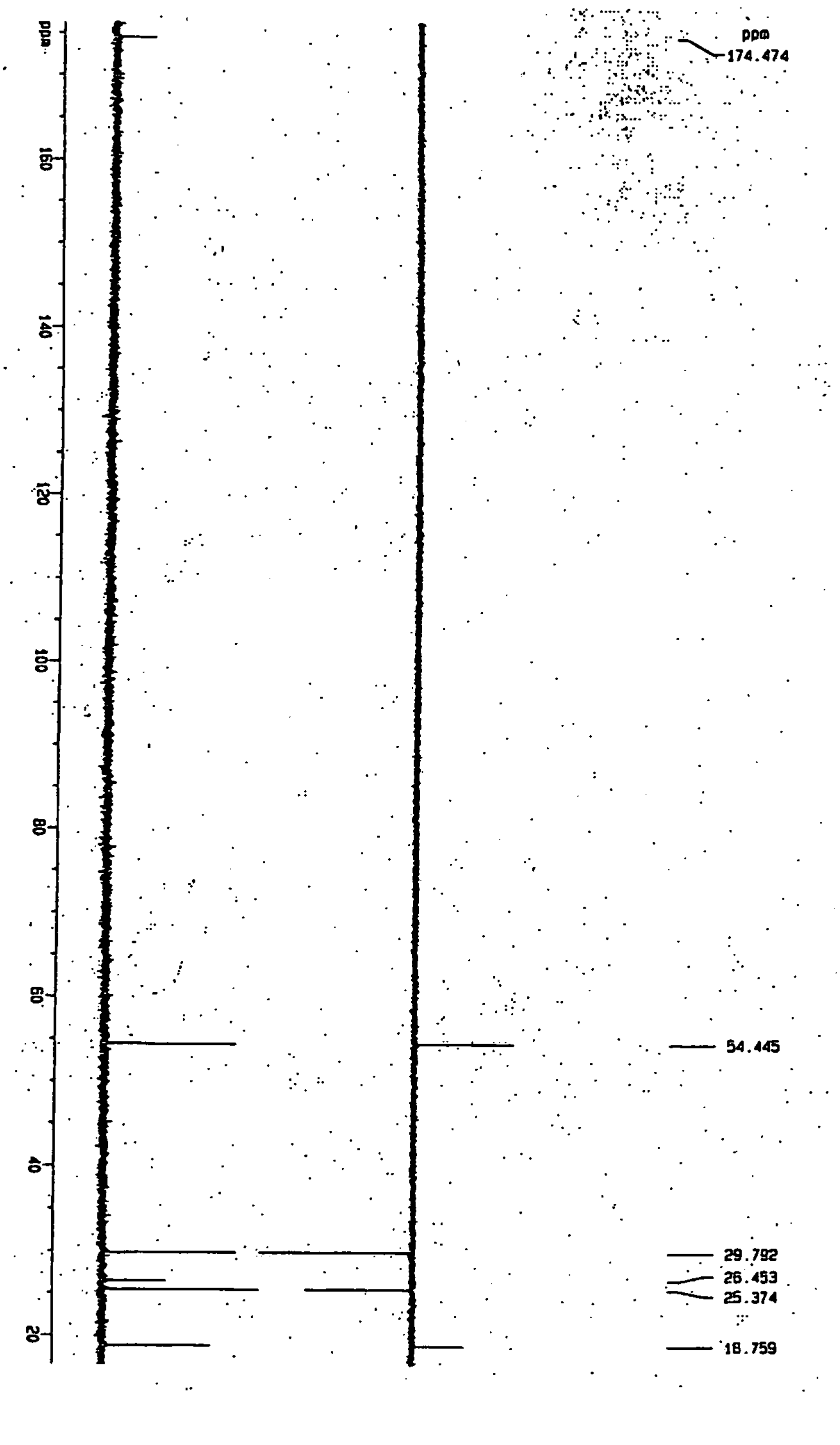
ppm
174.474
54.445
29.792
26.453
25.374
18.759
ppm
160
140
120
100
80
60
40
20

Figure 10

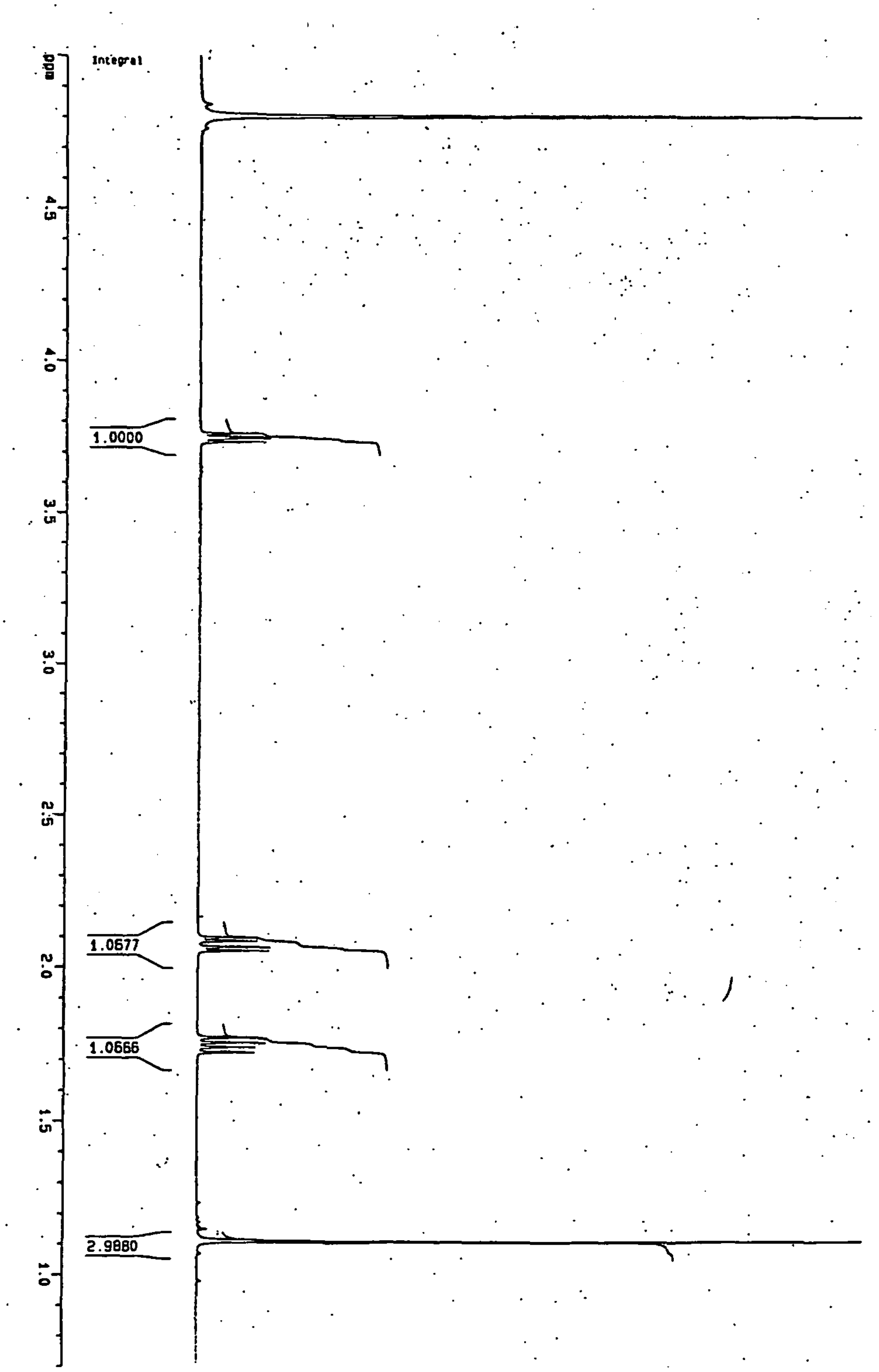

## Figure 11

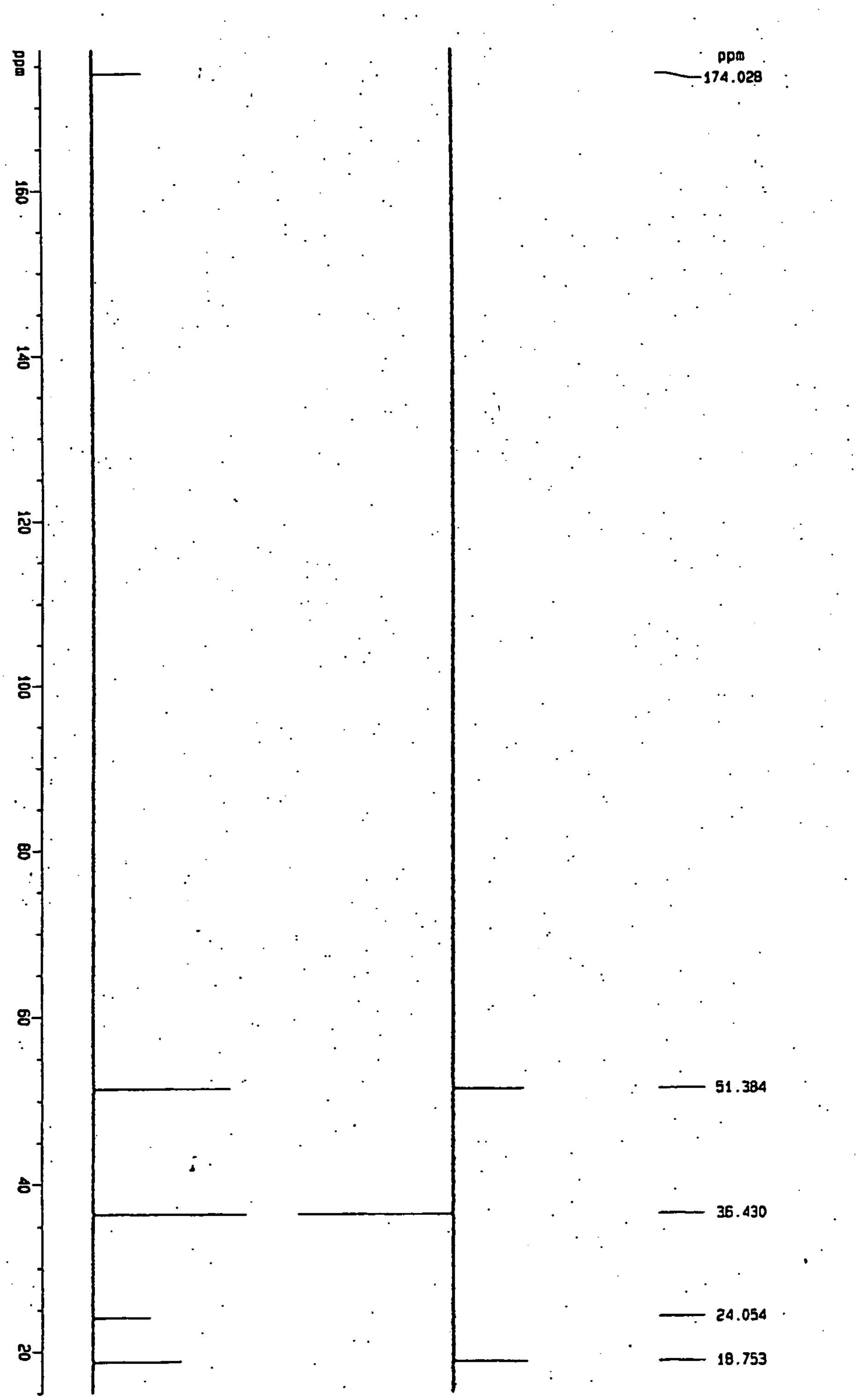
ppm
174.028
51.384
36.430
24.054
18.753
ppm
160
140
120
100
80
60
40
20

# Figure 12

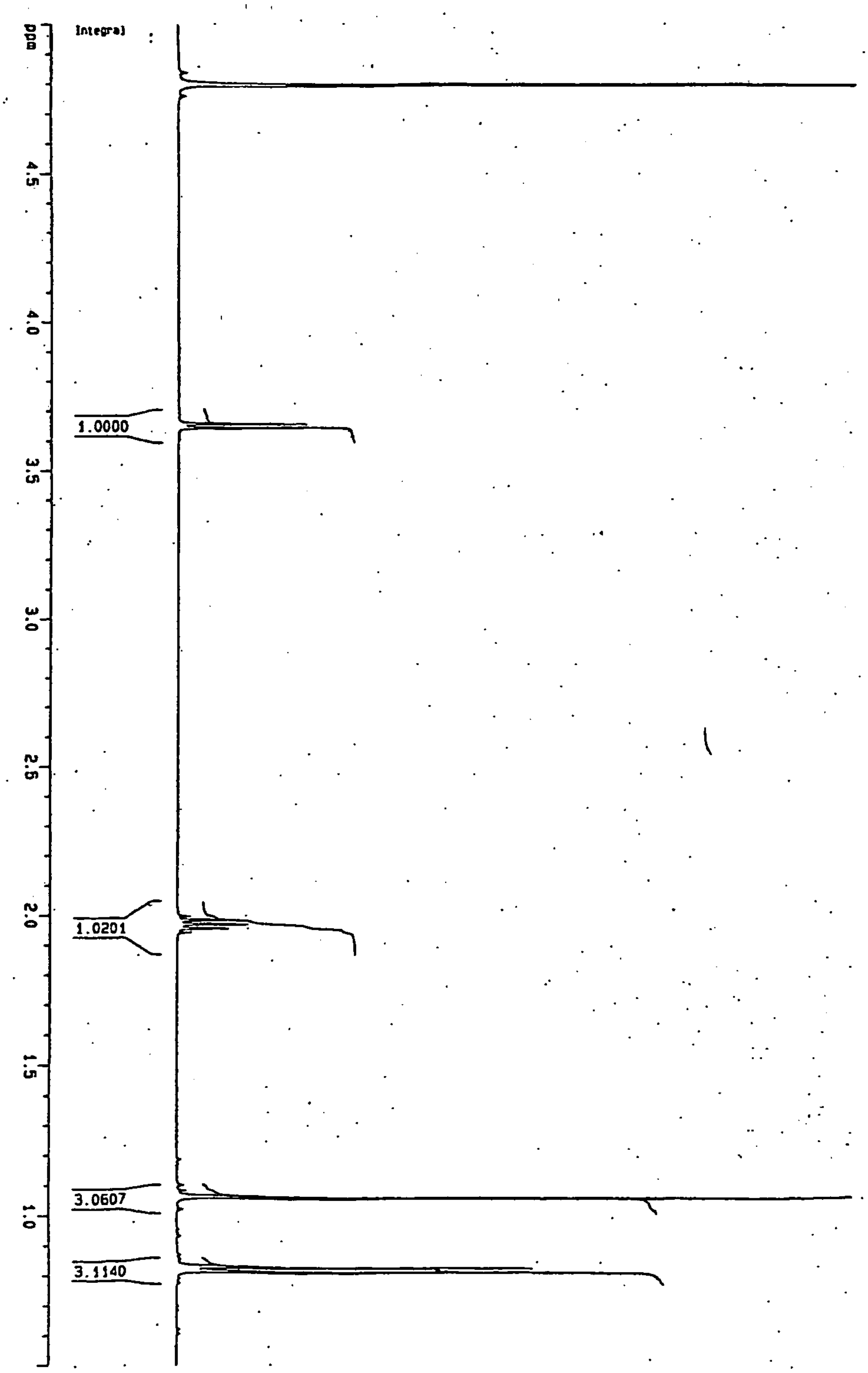

Figure 13

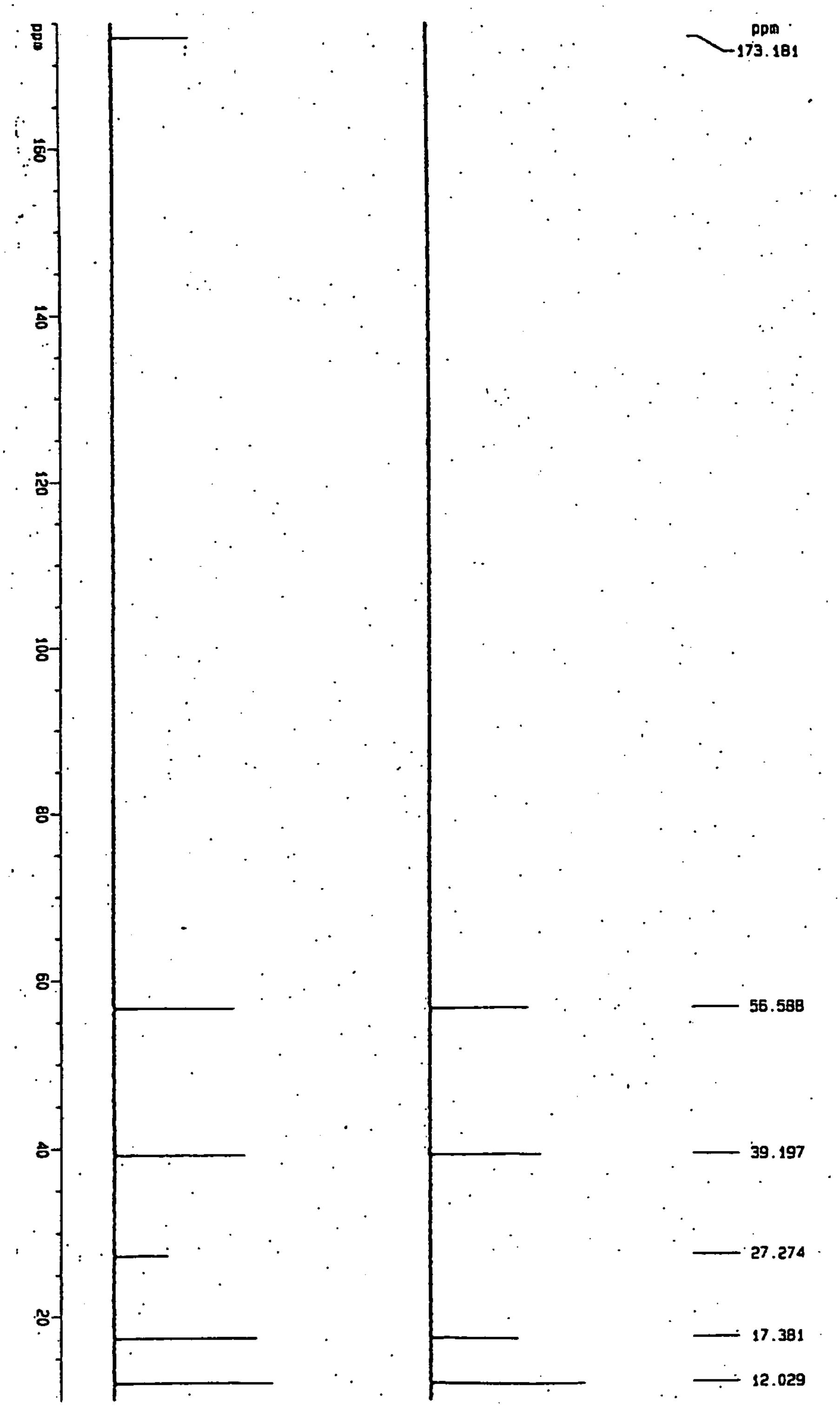
ppm
160
140
120
100
80
60
40
20
ppm
173.181
56.588
39.197
27.274
17.381
12.029

# Figure 14

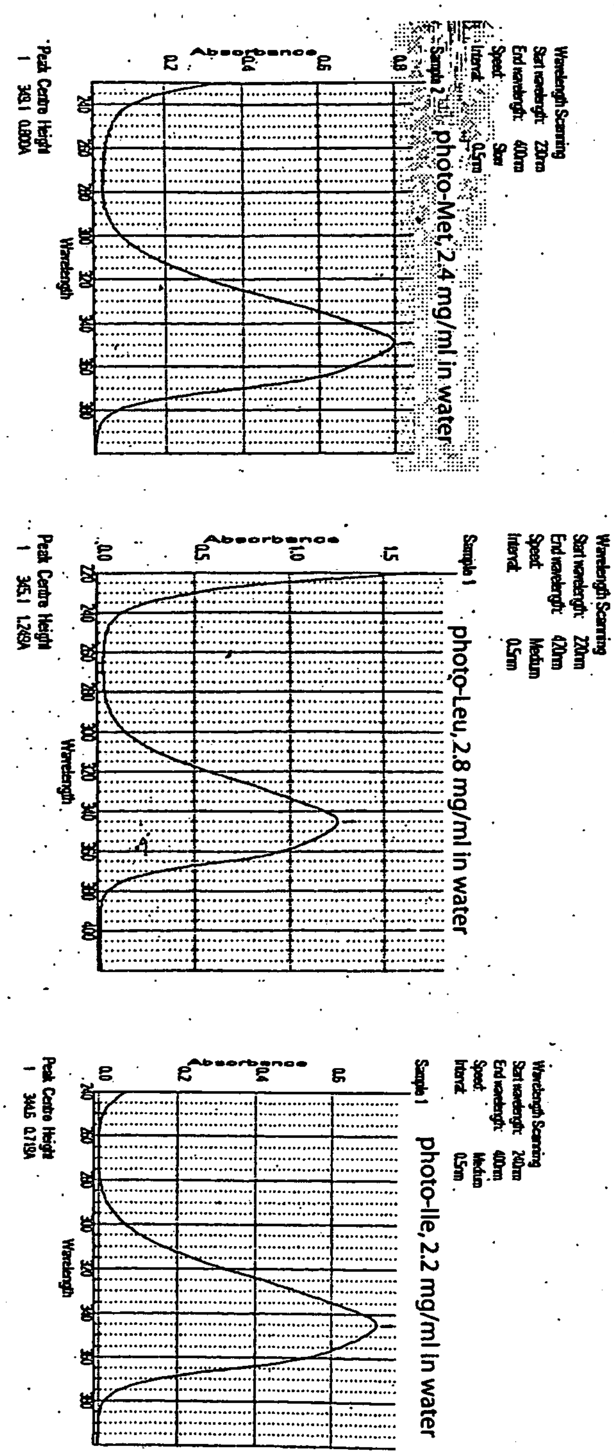
photo-Met, 2.4 mg/ml in water
photo-Leu, 2.8 mg/ml in water
photo-Ile, 2.2 mg/ml in water
Absorbance
Wavelength
Wavelength Scanning
Peak Centre Height

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Iwasa et al.** *Biosci. Biotech. Biochem.,* 1994, vol. 58 (5), 972-973
- **Ahmed et al.** *Biochem. J.,* 1992, vol. 286, 377-382
- **Fang et al.** *J. Am. Chem. Soc.,* 1998, vol. 120, 8543-8544
- **HO, Y. et al.** Systematic identification of protein complexes in Saccharomyces cerevisiae by mass spectrometry. *Nature,* 2002, vol. 415, 180-183
- **GAVIN, A.C. et al.** Functional organization of the yeast proteome by systematic analysis of protein complexes. *Nature,* 2002, vol. 415, 141-147
- **GADEK, T.R.** Strategies and methods in the identification of antagonists of protein-protein interactions. *Biotechniques,* 2003, 21-24
- **JI, T.H.** Bifunctional reagents. *Methods Enzymol,* 1983, vol. 91, 580-609
- **BAYLEY, H. ; KNOWLES, J.R.** Photoaffinity labeling. *Methods Enzymol.,* 1977, vol. 46, 69-114
- **JACOBS, S. ; HAZUM, E. ; SHECHTER, Y. ; CUATRECASAS, P.** Insulin receptor: covalent labeling and identification of subunits. *Proc Natl Acad Sci U S A,* 1979, vol. 76
- **THIELE, C. ; FAHRENHOLZ, F.** Photoaffinity labeling of central cholecystokinin receptors with high efficiency. *Biochemistry,* 1993, vol. 32, 2741-2746
- **KURZCHALIA, T.V. et al.** The signal sequence of nascent preprolactin interacts with the 54K polypeptide of the signal recognition particle. *Nature,* 1986, vol. 320, 634-636
- **KRIEG, U.C. ; WALTER, P. ; JOHNSON, A.E.** Photocrosslinking of the signal sequence of nascent preprolactin to the 54-kilodalton polypeptide of the signal recognition particle. *Proc Natl Acad Sci U S A,* 1986, vol. 83, 8604-8608
- **CORNISH, V.W. et al.** Site-specific incorporation of biophysical probes into proteins. *Proc Natl Acad Sci U S A,* 1994, vol. 91, 2910-2915
- **CHIN, J.W. ; MARTIN, A.B. ; KING, D.S. ; WANG, L. ; SCHULTZ, P.G.** Addition of a photocrosslinking amino acid to the genetic code of Escherichiacoli. *Proc Natl Acad Sci U S A,* 2002, vol. 99, 11020-11024
- **DEITERS, A. et al.** Adding amino acids with novel reactivity to the genetic code of Saccharomyces cerevisiae. *J Am Chem Soc,* 2003, vol. 125, 11782-11783
- **SAKAMOTO, K. et al.** Site-specific incorporation of an unnatural amino acid into proteins in mammalian cells. *Nucleic Acids Res,* 2002, vol. 30, 4692-4699
- **MONAHAN, S.L. ; LESTER, H.A. ; DOUGHERTY, D.A.** Site-specific incorporation of unnatural amino acids into receptors expressed in Mammalian cells. *Chem Biol,* 2003, vol. 10, 573-580
- **ZHANG, Z. et al.** Selective incorporation of 5-hydroxytryptophan into proteins in mammalian cells. *Proc Natl Acad Sci U S A,* 2004, vol. 101, 8882-8887
- **HARRP, D.N. ; BAO, L.Q. ; BLACK, C.J. ; GLEASON, J.G. ; SMITH, R.A.** An efficient alpha-halogenation of acyl chlorides by N-bromosuccinimide, N-chlorosuccinimide, and molecular iodine. *J. Org. Chem.,* 1975, vol. 40, 3420-3427
- **STRECKER, A.** Ueber die kuenstliche Bildung der Milchsaeure und eines neuen, dem Glycocoll homologen Koerper. *Liebigs Ann. Chem.,* vol. 75, 27-45
- **MOORE, S. ; STEIN, W.H.** Chromatography of amino acids on sulfonated polystyrene resins. *J Biol Chem,* 1951, vol. 192, 663-681
- **STENMARK, H. et al.** Inhibition of rab5 GTPase activity stimulates membrane fusion in endocytosis. *Embo J,* 1994, vol. 13, 1287-1296
- **SARGIACOMO, M. et al.** Oligomeric structure of caveolin: implications for caveolae membrane organization. *Proc Natl Acad Sci U S A,* 1995, vol. 92, 9407-9411
- **THIBONNIER, M.** The human platelet vasopressin receptor identification by direct ultraviolet photoaffinity labeling. *J Biol Chem,* 1987, vol. 262, 10960-10964
- **SNYERS, L. ; UMLAUF, E. ; PROHASKA, R.** Oligomeric nature of the integral membrane protein stomatin. *J Biol Chem,* 1998, vol. 273, 17221-17226
- **SIMONSEN, A. et al.** EEA1 links PI(3)K function to Rab5 regulation of endosome fusion. *Nature,* 1998, vol. 394, 494-498
- **FALKENSTEIN, E. ; MEYER, C. ; EISEN, C. ; SCRIBA, P.C. ; WEHLING, M.** Full-length cDNA sequence of a progesterone membrane-binding protein from porcine vascular smooth muscle cells. *Biochem Biophys Res Commun,* 1996, vol. 229, 86-89

- **KANAI, Y. et al.** Expression cloning and characterization of a transporter for large neutral amino acids activated by the heavy chain of 4F2 antigen (CD98. *J Biol Chem,* 1998, vol. 273, 23629-23632
- **FERRELL, K. ; WILKINSON, C.R. ; DUBIEL, W. ; GORDON, C.** Regulatory subunit interactions of the 26S proteasome, a complex problem. *Trends Biochem Sci,* 2000, vol. 25, 83-88
- **FU, H. ; REIS, N. ; LEE, Y. ; GLICKMAN, M.H. ; VIERSTRA, R.D.** Subunit interaction maps for the regulatory particle of the 26S proteasome and the COP9 signalosome. *Embo J,* 2001, vol. 20, 7096-7107
- **BROWN, M.S. ; GOLDSTEIN, J.L.** A proteolytic pathway that controls the cholesterol content of membranes, cells, and blood. *Proc Natl Acad Sci U S A,* 1999, vol. 96, 11041-11048
- **YANG, T. et al.** Crucial step in cholesterol homeostasis: sterols promote binding of SCAP to INSIG-1, a membrane protein that facilitates retention of SREBPs in ER. *Cell,* 2002, vol. 110, 489-500
- SREBPs: transcriptional mediators of lipid homeostasis. **HORTON, J.D. ; GOLDSTEIN, J.L. ; BROWN, M.S.** Cold Spring Harb Symp Quant Biol. 2002, vol. 67, 491-498
- **ADAMS, C.M. et al.** Cholesterol and 25-hydroxycholesterol inhibit activation of SREBPs by different mechanisms, both involving SCAP and insigs. *J Biol Chem,* 2004
- **NOLTE, I. ; JECKEL, D. ; WIELAND, F.T. ; SOHN, K.** Localization and topology of ratp28, a member of a novel family of putative steroid-binding proteins. *Biochim Biophys Acta,* 2000, vol. 1543, 123-130
- **THIELE, C. ; HANNAH, M.J. ; FAHRENHOLZ, F. ; HUTTNER, W.B.** Cholesterol binds to synaptophysin and is required for biogenesis of synaptic vesicles. *Nat Cell Biol,* 2000, vol. 2, 42-49
- **HENDRICKSON, T.L. ; DE CRECY-LAGARD, V. ; SCHIMMEL, P.** Incorporation of nonnatural amino acids into proteins. *Annu Rev Biochem,* 2004, vol. 73, 147-176
- **KIICK, K.L. ; WEBERSKIRCH, R. ; TIRRELL, D.A.** Identification of an expanded set of translationally active methionine analogues in Escherichia coli. *FEBS Lett,* 2001, vol. 502, 25-30
- **LINK, A.J. ; MOCK, M.L. ; TIRRELL, D.A.** Non-canonical amino acids in protein engineering. *Curr Opin Biotechnol,* 2003, vol. 14, 603-609
- **RENNERT, O.M. ; ANKER, H.S.** On the Incorporation of 5',5',5'-Trifluoroleucine into Proteins of E. Coli. *Biochemistry,* 1963, vol. 13, 471-476
- **DORING, V. et al.** Enlarging the amino acid set of Escherichia coli by infiltration of the valine coding pathway. *Science,* 2001, vol. 292, 501-504
- **MURSINNA, R.S. ; MARTINIS, S.A.** Rational design to block amino acid editing of a tRNA synthetase. *J Am Chem Soc,* 2002, vol. 124, 7286-7287
- **TANG, Y. ; TIRRELL, D.A.** Attenuation of the editing activity of the Escherichia coli leucyl-tRNA synthetase allows incorporation of novel amino acids into proteins in vivo. *Biochemistry,* 2002, vol. 41, 10635-10645
- **JONES, S. ; THORNTON, J.M.** Principles of protein-protein interactions. *Proc Natl Acad Sci U S A,* 1996, vol. 93, 13-20
- **DUPREE, P. ; PARTON, R.G. ; RAPOSO, G. ; KURZCHALIA, T.V. ; SIMONS, K.** Caveolae and sorting in the trans-Golgi network of epithelial cells. *Embo J,* 1993, vol. 12, 1597-1605
- **WESSEL, D. ; FLUEGGE, U.I.** A method for the quantitative recovery of protein in dilute solution in the presence of detergents and lipids. *Anal. Biochem.,* 1984, vol. 138, 141-143
- **UDENFRIEND, S. et al.** Fluorescamine: a reagent for assay of amino acids, peptides, proteins, and primary amines in the picomole range. *Science,* 1972, vol. 178, 871-872
- **TOURASSE, N.J. ; LI, W.H.** Selective constraints, amino acid composition, and the rate of protein evolution. *Mol Biol Evol,* 2000, vol. 17, 656-664
- **BRUNNER, J.** New photolabeling and crosslinking methods. *Annu. Rev. Biochem.,* 1993, vol. 62, 483-514
- **CHURCH, R.F.R. ; WEISS, M.J.** Diazirines. II. Synthesis and properties of small functionalized diazirine molecules. Some observations on the reaction of a diaziridine with the iodine-iodide ion system. *J. Org. Chem.,* 1970, vol. 35, 2465-2471
- **HARRP, D.N. ; BAO, L.Q. ; BLACK, C.J. ; GLEASON, J.G. ; SMITH, R.A.** An efficient alpha-halogenation of acyl chlorides by N-bromosuccinimide, N-chlorosuccinimide, and molecular iodine. *J. Org. Chem.,* 1975, vol. 40, 3420-3427
- **HENDRICKSON, T. L. ; NOMANBHOY, T. K. ; SCHIMMEL, P.** Errors from selective disruption of the editing center in a tRNA synthetase. *Biochemistry,* 2000, vol. 39, 8180-8186
- **LEE, J.W. ; LEE, S.K.** Mammalian two-hybrid assay for detecting protein-protein interactions in vivo. *Methods Mol Biol,* 2004, vol. 261, 327-336
- **MILLER, J. ; STAGLJAR, I.** Using the yeast two-hybrid system to identify interacting proteins. *Methods Mol Biol,* 2004, vol. 261, 247-262
- **NASSAL, M.** 4'-(1-Azi-2,2,2-trifluoroethyl)phenylalanine, a photolabile carbene-generating analogue of phenylalanine. *J. Amer. Chem. Soc.,* 1984, vol. 106, 7540-7545
- **SANTORO, S. W. ; WANG, L. ; HERBERICH, B. ; KING, D. S. ; SCHULTZ, P. G.** An efficient system for the evolution of aminoacyl-tRNA synthetase specificity. *Nature Biotech.,* 2002, vol. 20, 1044-1048

- **THAMINY, S. ; MILLER, J. ; STAGLJAR, I.** The split-ubiquitin membrane-based yeast two-hybrid system. *Methods Mol Biol,* 2004, vol. 261, 297-312
- **TURRO, N. J. ; CHAN, Y. ; GOULD, I. R.** Reactivity and intersystem crossing of singlet methylene in solution. *J. Amer. Chem. Soc.,* 1987, vol. 109, 2101-2107